(19) Europäisches Patentamt
European Patent Office
Office européen des brevets

(11) EP 0 215 594 B2

(12) NEW EUROPEAN PATENT SPECIFICATION

(45) Date of publication and mention of the opposition decision:
**15.10.2003 Bulletin 2003/42**

(45) Mention of the grant of the patent:
**11.01.1995 Bulletin 1995/02**

(21) Application number: **86306624.7**

(22) Date of filing: **27.08.1986**

(51) Int Cl.[7]: **C12N 15/00**, C12N 1/14, C12P 21/02 // (C12N1/14, C12R1:66, 1:665, 1:785, 1:885)

# (54) Heterologous polypeptide expressed in filamentous fungi, processes for their preparation, and vectors for their preparation

Expression von heterologen Polypeptiden in filamentösen Pilzen, Verfahren zu deren Herstellung und Vektoren zu deren Herstellung

Polypeptides hétérologues exprimés dans les champignons filamenteux, leur procédé de préparation ainsi que vecteurs pour leur préparation

(84) Designated Contracting States:
**AT BE CH DE FR GB IT LI LU NL SE**

(30) Priority: **29.08.1985 US 771374**
**07.07.1986 US 882224**

(43) Date of publication of application:
**25.03.1987 Bulletin 1987/13**

(60) Divisional application:
**94201751.8 / 0 625 577**

(73) Proprietor: **GENENCOR INTERNATIONAL, INC.**
**South San Francisco, CA 94080 (US)**

(72) Inventors:
- **Berka, Randy M.**
**No. 5 San Mateo California 94403 (US)**
- **Cullen, Daniel**
**Madison, Wisconsin 53711 (US)**
- **Gray, Gregory L.**
**South San Francisco California 94980 (US)**
- **Hayenga, Kirk J.**
**Burlingame California 94010 (US)**
- **Lawlis, Virgil B.**
**San Mateo, California 94403 (US)**

(74) Representative: **Armitage, Ian Michael et al**
**MEWBURN ELLIS**
**York House**
**23 Kingsway**
**London WC2B 6HP (GB)**

(56) References cited:
**EP-A- 0 126 206**
**EP-A- 0 238 023**
**EP-A- 0 249 350**
**WO-A-86/06097**

- **PROCEEDINGS OF THE NATIONAL ACADEMY OF SCIENCE, vol. 81, March 1984, pages 1470-1474, Washington, US; M.M. YELTON et al.: "Transformation of aspergillus nidulans by using a trpC plasmid"**
- **GENE, vol. 36, no. 3, 1985, pages 321-331, Elsevier Science Publishers, Amsterdam, NL; D.J. BALLANCE et al.: "Development of a high-frequency transforming vector for Aspergillus nidulans"**
- **JOURNAL OF CELLULAR BIOCHEMISTRY SUPPL., vol. 0, no. 10, part A, February 1986, Los Angeles, US; K. HAYENGA: "Expression and secretion of bovine calf chymosinby Aspergillus nidulans"**
- **HEREDITY, vol. 57, no. 1, June/July 1986, page 131, Edinburgh, Scotland, GB; D.CULLEN et al.: "Expression of bovine chymosin by Aspergillus nidulans"**
- **NETHERLANDS MILK DAIRY JOURNAL, vol. 35, 1981, pages 275-280, Wageningen, NL;A.-M. BECH et al.: "Partial primary structure of Mucor miehei protease"**
- **CHEMICAL ABSTRACTS, vol. 98, no. 15, April 1983, page 173, abstract no.120575m, Columbus, Ohio, US; D. MOIR et al.: "Molecular cloning and characterisation of double-stranded cDNA coding for bovine chymosin"**
- **Alexopoulos, C.J. 1962, Introductory Mycology, pp. 100-101, John Wiley and Sons, Inc., New York.**

Remarks:
Divisional application 94201751.8 filed on 27/08/86.



Printed by Jouve, 75001 PARIS (FR)

## Description

The present invention is concerned with heterologous polypeptides expressed and secreted by filamentous fungi and with vectors and processes for expressing and secreting such polypeptides. More particularly, the invention discloses transformation vectors and, in particular, processes using the same for expressing and secreting biologically active bovine chymosin and heterologous glucoamylase by a filamentous fungus.

The expression of DNA sequences encoding heterologous polypeptides (i.e., polypeptides not normally expressed and secreted by a host organism) has advanced to a state of considerable sophistication. For example, it has been reported that various DNA sequences encoding pharmacologically desirable polypeptides [e.g., human growth hormone (1), human tissue plasminogen activator (2), various human interferons (6), urokinase (5), Factor VIII (4), and human serum albumin (3)] and industrially important enzymes [e.g., chymosin (7), alpha amylases (8), and alkaline proteases (9)] have been cloned and expressed in a number of different expression hosts. Such expression has been achieved by transforming prokaryotic organisms [e.g., E. coli (10) or B. subtilis (11)] or eukaryotic organisms [e.g., Saccharomyces cerevisiae (7), Kluyveromyces lactis (12) or Chinese Hamster Ovary cells (2)] with DNA sequences encoding the heterologous polypeptide.

Some polypeptides, when expressed in heterologous hosts, do not have the same level of biological activity as their naturally produced counterparts when expressed in various host organisms. For example, bovine chymosin has very low biological activity when expressed by E. coli (13) or S. cerevisiae (7). This reduced biological activity in E. coli is not due to the natural inability of E. coli to glycosylate the polypeptide since chymosin is not normally glycosylated (14). Such relative inactivity, both in E. coli and S. cerevisiae, however, appears to be primarily due to improper folding of the polypeptide chain as evidenced by the partial post expression activation of such expressed polypeptides by various procedures. In such procedures, expressed chymosin may be sequentially denatured and renatured in a number of ways to increase biological activity: e.g., treatment with urea (13), exposure to denaturing/renaturing pH (13) and denaturation and cleavage of disulfide bonds followed by renaturation and regeneration of covalent sulfur linkages (15). Such denaturation/renaturation procedures, however, are not highly efficient [e.g., 30% or less recovery of biological activity for rennin (13)], and add considerable time and expense in producing a biologically active polypeptide.

Other heterologous polypeptides are preferably expressed in higher eukaryotic hosts (e.g., mammalian cells). Such polypeptides are usually glycopolypeptides which require an expression host which can recognize and glycosylate certain amino acid sequences in the heterologous polypeptide. Such mammalian tissue culture systems, however, often do not secrete large amounts of heterologous polypeptides when compared with microbial systems. Moreover, such systems are technically difficult to maintain and consequently are expensive to operate.

Transformation and expression in a filamentous fungus involving complementation of aroD mutants of N. crassa lacking biosynthetic dehydroquinase has been reported (16). Since then, transformation based on complementation of glutamate dehydrogenase deficient N. crassa mutants has also been developed (17). In each case the dehydroquinase (ga2) and glutamate dehydrogenase (am) genes used for complementation were derived from N. crassa and therefore involved homologous expression. Other examples of homologous expression in filamentous fungi include the complementation of the auxotrophic markers trpC, (18) and argB (19) in A. nidulans and the transformation of A. nidulans to acetamide or acrylamide utilization by expression of the A. nidulans gene encoding acetamidase (20).

Expression of heterologous polypeptides in filamentous fungi has been limited to the transformation and expression of fungal and bacterial polypeptides. For example, A. nidulans, deficient in orotidine-5'-phosphate decarboxylase, has been transformed with a plasmid containing DNA sequences encoding the pyr4 gene derived from N. crassa (21,32). A. niger has also been transformed to utilize acetamide and acrylamide by expression of the gene encoding acetamidase derived from A. nidulans (22).

Examples of heterologous expression of bacterial polypeptides in filamentous fungi include the expression of a bacterial phosphotransferase in N. crassa (23) Dictyostellium discoideum (24) and Cephalosporium acremonium (25).

In each of these examples of homologous and heterologous fungal expression, the expressed polypeptides were maintained intracellularly in the filamentous fungi.

Accordingly, an object of the invention herein is to provide for the expression and secretion of heterologous polypeptides by and from filamentous fungi including vectors for transforming such fungi and processes for expressing and secreting such heterologous polypeptides.

The invention is more particulary defined in the appended claims.

Brief Description of the Drawings

Figure 1 is a restriction map of the Aspergillus niger glucoamylase inserts in pGa1 and pGa5.
Figure 2 depicts the construction of pDJB-gam-1.
Figure 3 depicts the construction of mp19GAPR.
Figure 4, 5, 6, and 7 depict the construction of pGRG1, pGRG2, pGRG3, and pGRG4.
Figure 8 shows the strategy used to generate mp19 GAPR$^{\Delta}$CI - $^{\Delta}$C4 from mp19 GAPR.
Figure 9 depicts the construction of pCR160.
Figure 10 is a partial restriction map of the Mucor miehei carboxyl protease gene including 5' and 3' flanking sequences.
Figures 11 A.B. and C is the DNA sequence of Mucor miehei, carboxyl protease including the entire coding sequence and 5' and 3' flanking sequences.
Figure 12 depicts the construction of pMeJB1-7.
Figures 13 A and B are a partial nucleotide and restriction map of ANS-1.
Figure 14 depicts the construction of pDJB-3
Figure 15 depicts the construction of plasmid pCJ:GRG1 through pCJ:GRG4.
Figure 16 depicts a restriction endonuclease cleavage map of the 3.7Kb BamHI fragment from pRSHI.
Figure 17 depicts the construction of pCJ:RSH1 and pCJ:RSH2.
Figure 18 depicts the expression of H. grisea glucoamylase from A. nidulans.

Detailed Description

The inventors have demonstrated that heterologous polypeptides from widely divergent sources can be expressed and secreted by filamentous fungi. Specifically, bovine chymosin, glucoamylase from Aspergillus niger and Humicola grises and the carboxyl protease from Mucor miehei have been expressed in and secreted from A. nidulans. In addition, bovine chymosin has been expressed and secreted from A. awamori and Trichoderma reesei. Biologically active chymosin was detected in the culture medium without further treatment. This result was surprising in that the vectors used to transform A. nidulans were constructed to secrete prochymosin which requires exposure to an acidic environment (approximately pH 2) to produce biologically active chymosin.
In general, a vector containing DNA sequences encoding functional promoter and terminator sequences (including polyadenylation sequences) are operably linked to DNA sequences encoding various signal sequences and heterologous polypeptides. The thus constructed vectors are used to transform a filamentous fungus. viable transformants may thereafter be identified by screening for the expression and secretion of the heterologous polypeptide.
Alternatively, an expressible selection characteristic may be used to isolate transformants by incorporating DNA sequences encoding the selection characteristic into the transformation vector. Examples of such selection characteristics include resistance to various antibiotics, (e.g., aminoglycosides, benomyl etc.) and sequences encoding genes which complement an auxotrophic defect (e.g. pyr4 complementation of pyr4 deficient A. nidulans, A. awamori or Trichoderma reesei or ArgB complementation of ArgB deficient A. nidulans or A. awamori) or sequences encoding genes which confer a nutritional (e.g., acetamidase) or morphological marker in the expression host.
In the preferred embodiments disclosed a DNA sequence encoding the ANS-1 sequence derived from A. nidulans is included in the construction of the transformation vectors of the present invention. This sequence increases the transformation efficiency of the vector. Such sequences, however, are not considered to be absolutely necessary to practice the Invention.
In addition, certain DNA sequences derived from the bacterial plasmid pBR325 form part of the disclosed transformation vectors. These sequences also are not believed to be necessary for transforming filamentous fungi. These sequences instead provide for bacterial replication of the vectors during vector construction. Other plasmid sequences which may also be used during vector construction include pBR322 (ATCC 37017), RK-2 (ATCC 37125), pMB9 (ATCC 37019) and pSC101 (ATCC 37032).
The disclosed preferred embodiments are presented by way of example and are not intended to limit the scope of the invention.

Definitions

By "expressing polypeptides" is meant the expression of DNA sequences encoding the polypeptide.
"Polypeptides" are polymers of $\alpha$-amino acids which are covalently linked through peptide bonds. Polypeptides include low molecular weight polymers as well as high molecular weight polymers more commonly referred to as proteins. In addition, a polypeptide can be a phosphopolypeptide, glycopolypeptide or metallopolypeptide. Further, one or more polymer chains may be combined to form a polypeptide.

As used herein a "heterologous polypeptide" is a polypeptide which is not normally expressed and secreted by the filamentous fungus used to express that particular polypeptide. Heterologous polypeptides include polypeptides derived from prokaryotic sources (e.g., α-amylase from Bacillus species, alkaline protease from Bacillus species, and various hydrolytic enzymes from Pseudomonas, etc.), polypeptides derived from eukaryotic sources (e.g., bovine chymosin, human tissue plasminogen activator, human growth hormone, human interferon, urokinase, human serum albumin, factor VIII etc.), and polypeptides derived from fungal sources other than the expression host (e.g., glucoamylase from A. niger and Humicola grisea expressed in A. nidulans, the carboxyl protease from Mucor miehei expressed in A. nidulans, etc.).

Heterologous polypeptides also include hybrid polypeptides which comprise a combination of partial or complete polypeptide sequences derived from at least two different polypeptides each of which may be homologous or heterologous with regard to the fungal expression host. Examples of such hybrid polypeptides include DNA sequences encoding fungal glucoamylase or any fungal carboxy protease, human tissue plasminogen activator or human growth hormone fused to DNA sequences encoding the functional signal sequence alone or in conjunction with various amounts of amino-terminal propeptide condons or mature codons associated with the functional signal.

Further, the heterologous polypeptides of the present invention also include: 1) naturally occuring allellic variations that may exist or occur in the sequence of polypeptides derived from the above prokaryotic, eukaryotic and fungal sources as well as those used to form the above hybrid polypeptides, and 2) engineered variations in the above heterologous polypeptides brought about, for example, by way of site specific mutagenesis wherein various deletions, insertions or substitutions of one or more of the amino acids in the heterologous polypeptides are produced.

A "biochemically active heterologous polypeptide" is a heterologous polypeptide which is secreted in active form as evidenced by its ability to mediate: 1) the biochemical activity mediated by its naturally occurring counterpart, or 2) in the case of hybrid polypeptides, the biochemical activity mediated by at least one of the naturally occurring counterparts comprising the hybrid polypeptides.

Each of the above defined heterologous polypeptides is encoded by a heterologous DNA sequence which contains a stop signal which is recognized by the filamentous fungus in which expression and secretion occurs. When recognized by the host, the stop signal terminates translation of the mRNA encoding the heterologous polypeptide.

The "filamentous fungi" of the present invention are eukaryotic microorganisms and include all filamentous forms of the subdivision Eumycotina (26). These fungi are characterized by a vegatative mycelium composed of chitin, cellulose, and other complex polysaccharides. The filamentous fungi of the present invention are morphologically, physiologically, and genetically distinct from yeasts. Vegetative growth by filamentous fungi is by hyphal elongation and carbon catabolism is obligately aerobic. In contrast, vegetative growth by yeasts such as S. cerevisiae is by budding of a unicellular thallus, and carbon catabolism may be fermentative. S cerevisiae has a prominent, very stable diploid phase whereas, diploids exist only briefly prior to meiosis in filamentous fungi like Aspergilli and Neurospora. S. cervisiae has 17 chromosomes as opposed to 8 and 7 for A. nidulans and N. crassa respectively. Recent illustrations of differences between S. cerevisiae and filamentous fungi include the inability of S. cerevisiae to process Aspergillus and Trichoderma introns and the inability to recognize many transcriptional regulators of filamentous fungi (27).

Various species of filamentous fungi may be used as expression hosts including the following genera: Aspergillus, Trichoderma, Neurospora, Podospora, Endothia Mucor, Cochiobolus and Pyricularia. Specific expression hosts include A. nidulans (18, 19, 20, 21, 61), A. niger (22), A. awomari, e.g., NRRL 3112, ATCC 22342 (NRRL 3112), ATCC 44733, ATCC 14331 and strain UVK 143f, A. oryzae, e.g., ATCC 11490, N. crassa (16, 17, 23), Trichoderma reesei, e.g. NRRL 15709, ATCC 13631, 56764, 56765, 56466, 56767, and Trichoderma viride, e.g., ATCC 32098 and 32086.

As used herein, a "promotor sequence" is a DNA sequence which is recognized by the particular filamentous fungus for expression purposes. It is operably linked to a DNA sequence encoding the above defined polypeptides. Such linkage comprises positioning of the promoter with respect to the initiation codon of the DNA sequence encoding the signal sequence of the disclosed transformation vectors. The promoter sequence contains transcription and translation control sequences which mediate the expression of the signal sequence and heterologous polypeptide. Examples include the promoter from A. niger glucoamylase (39,48), the Mucor miehei carboxyl protease herein, and A. niger α-glucosidase (28), Trichoderma reesei cellobiohydrolase I (29), A. nidulans trpC (18) and higher eukaryotic promoters such as the SV40 early promoter (24).

Likewise a "terminator sequence" is a DNA sequence which is recognized by the expression host to terminate transcription. It is operably linked to the 3' end of the DNA encoding the heterologous polypeptide to be expressed. Examples include the terminator from A. nidulans trpC (18), A. niger glucoamylase (39,48), A. niger α-glucosidase (28), and the Mucor miehei carboxyl protease herein, although any fungal terminator is likely to be functional in the present invention.

A "polyadenylation sequence" is a DNA sequence which when transcribed is recognized by the expression host to add polyadenosine residues to transcribed mRNA. It is operably linked to the 3' end of the DNA encoding the heterologous polypeptide to be expressed. Examples include polyadenylation sequences from A. nidulans trpC (18), A. niger glucoamylase (39,48), A. niger α-glucosidase (28), and the Mucor miehei carboxyl protease herein. Any fungal polyadenytation sequence, however, is likely to be functional in the present invention.

A "signal sequence" is an amino acid sequence which when operably linked to the amino-terminus of a heterologous polypeptide permits the secretion of such heterologus polypeptide from the host filamentous fungus. Such signal sequences may be the signal sequence normally associated with the heterologous (mammalian) polypeptide (i. e., a native signal sequence) or comprises the signal sequence of bovine preprochymosin (15) or Mucor miehei preprocarboxy protease. Signal sequences are operably linked to a heterologous polypeptide either by utilizing the native signal sequence or by joining a DNA sequence encoding the signal sequence to a DNA sequence encoding the heterologous polypeptide in the proper reading frame to permit translation of the signal sequence and heterologous polypeptide.

A "propeptide" or "pro sequence" is an amino acid sequence positioned at the amino terminus of a mature biologically active polypeptide. When so positioned the resultant polypeptide is called a zymogen. Zymogens, generally, are biologically inactive and can be converted to mature active polypeptides by catalytic or autocatalytic cleavage of the propeptide from the zymogen.

In one embodiment of the invention a "transformation vector" is a DNA sequence encoding a heterologous polypeptide and a DNA sequence encoding a heterologous or homologous signal sequence operably linked thereto. In addition, a transformation vector may include DNA sequences encoding functional promoter and polyadenylation sequences. Each of the above transformation vectors may also include sequences encoding an expressible selection characteristic as well as sequences which increase the efficiency of fungal transformation.

"Transformation" is a process wherein a transformation vector is introduced into a filamentous fungus. The methods of transformation of the present invention have resulted in the stable integration of all or part of the transformation vector into the genome of the filamentous fungus. However, transformation resulting in the maintenance of a self-replicating extrachromosomal transformation vector is also contemplated.

General Methods

"Digestion" of DNA refers to catalytic cleavage of the DNA with an enzyme that acts only at certain locations in the DNA. Such enzymes are called restriction enzymes, and the sites for which each is specific is called a restriction site. "Partial" digestion refers to incomplete digestion by a restriction enzyme, i.e., conditions are chosen that result in cleavage of some but not all of the sites for a given restriction endonuclease in a DNA substrate. The various restriction enzymes used herein are commercially available and their reaction conditions, cofactors and other requirements as established by the enzyme suppliers were used. In general, about 1 microgram of plasmid or DNA fragment is used with about 1 unit of enzyme and about 20 microliters of buffer solution. Appropriate buffers and substrate amounts with particular restriction enzymes are specified by the manufacturer. Incubation times of about one hour at 37°C are ordinarily used, but may vary in accordance with the supplier's instructions. After incubation, protein is removed by extraction with phenol and chloroform, and the digested nucleic acid is recovered from the aqueous fraction by precipitation with ethanol. Digestion with a restriction enzyme may be followed by bacterial alkaline phosphatase hydrolysis of the terminal 5' phosphates to prevent the two ends of a DNA fragment from forming a closed loop that would impede insertion of another DNA fragment at the restriction site upon ligation.

"Recovery" or "isolation" of a given fragment of DNA from a restriction digest means separation of the digest by a polyacrylamide gel electrophoresis, identification of the fragment of interest, removal of the gel section containing the desired fragment, and separation of the DNA from the gel generally by electroelution (30).

"Ligation" refers to the process of forming phosphodiester bonds between two double-stranded nucleic acid fragments (30). Unless otherwise stated, ligation was accomplished using known buffers in conditions with one unit of T4 DNA ligase ("ligase") per 0.5 microgram of approximately equal molar amounts of the DNA fragments to be ligated.

"Oligonucleotides" are short length single or double stranded polydeoxynucleotides which were chemically synthesized by the method of Crea et al., (31) and then purified on polyacrylamide gels.

"Transformation" means introducing DNA in to an organism so that the DNA is maintained, either as an extrachromosomal element or chromosomal integrant. Unless otherwise stated, the method used herein for transformation of E. coli was the $CaCl_2$ method (30).

A. nidulans strain G191 (University of Glasgow culture collection) was transformed by incubating A. nidulans sphaeroplasts with the transformation vector. The genotype of strain G191 is pabaA1 (requires p-aminobenzoic acid), fwA1 (a color marker), mauA2 (monoamine non-utilizing), and pyrG89 (deficient for orotidine phosphate decarboxlase). Sphaeroplasts were prepared by the cellophane method of Ballance et al. (21) with the following modifications. To digest A. nidulans cell walls, Novozyme 234 (Novo Industries, Denmark) was first partially purified. A 100 to 500 mg sample of Novozyme 234 was dissolved in 2.5 ml of 0.6M KCl. The 2.5 ml aliquot was loaded into a PD10 column (Pharmacia-Upsulla, Sweden) equilibrated with 0.6M KCl. The enzymes were eluted with 3.5 ml of the same buffer.

Cellophane discs were incubated in Novozyme 234 (5 mg/ml) for 2 hours, then washed with 0.6M KCl. The digest and washings were combined, filtered through miracloth (Calbiochem-Behring Corp., La Jolla, CA), and washed as described (21). Centrifugations were in 50 or 15 ml conical tubes at ca. 1000Xg for 10 min. Following incubation on ice for 20 min, 2 ml of the polyethylene glycol 4000 solution (250 mg/ml) was added, incubated at room temperature for 5 min. followed by the addition of 4 ml of 0.6M KCl, 50mM $CaCl_2$. Transformed protoplasts were centrifuged, resuspended in 0.6M KCl, 50mM $CaCl_2$, and plated as described (21). Zero controls comprised protoplasts incubated with 20 μl of 20mM Tris-HCl, 1mM EDTA, pH7.4 without plasmid DNA. Positive controls comprised transformation with 5μg of pDJB3 constructed as described herein. Regeneration frequencies were determined by plating dilutions on minimal media supplemented with 5-10ppm paba and 500ppm uridine. Regeneration ranged from 0.5 to 5%.

Because of the low transformation frequencies associated with pDJB1, the derivative containing the Mucor acid protease gene (pMeJB1-7) was expected to give extremely low transformation frequencies. Consequently, to obtain pmeJB|1-7 transformants of A. nidulans, cotransformation was used. This was accomplished by first constructing a non-selectable vector containing ANS-1, and then transforming sphaeroplasts with a mixture of pmeJB1-7 and the non-selectable vector containing the ANS-1 fragment. The rationale for this approach was that the ANS-1 bearing vector would integrate in multiple copies and provide regions of homology for pMeJB1-7 integration. The ANS-1 vector was prepared by subcloning the PstI-PvuII fragment of ANS-1 (Figure 12A and 13B) from pDJB-3 into pUC18 (33).

The two plasmids (pMeJB1-7 and the ANS-1 containing vector) were mixed (2.5 μg each) and the above mentioned transformation protocol followed.

Transformants obtained with vectors PGRG1-pGRG4 and pDJB-gam were transferred after 3 or 4 days incubation at 37° C. Minimal media agar plates supplemented with 5 ppm p-aminobenzoic acid were centrally inoculated with mycelial transfers from transformants. Three to five days following inoculation of minimal medium plates, spore suspensions were prepared by vortexing a mycelial fragment in Iml distilled $H_2O$, 0.02% tween-80. Approximately $5 \times 10^4$ spores were inoculated into 250 ml baffled flasks containing 50 ml of the following medium: (g/l) Maltodextrin M-040 (Grain Processing Corp., Muscatine, Iowa) 50g, $NaNO_3$ 6g, $MgSO_4$ .7H20 0.5g, KCl 0.52g, $KH_2PO_4$, 68g, 1ml trace element solution (34), 1ml MAZU DF-60P antifoam (Mazer Chemicals, Inc., Gurnee, IL), 10 ppm p-aminobenzoic acid, and 50 ppm streptomycin sulfate. Alternatives to MAZU, such as bovine serum albumin or other appropriate surfactant may be used. Mucor acid protease secretion was tested in Aspergillus complete medium (20g dextrose, 1g peptone, 20g malt extract per liter). Carbon source regulation of chymosin secretion by Aspergillus nidulans transformants was assessed by measuring secretion in the above-mentioned starch medium relative to the same medium supplemented with 1% fructose, sucrose, or dextrose instead of 5% starch. In all cases, the media were incubated at 37 °C on a rotary shaker (150rpm). A pDJB3-derived transformant was included as a control.

Western blots of the various secreted chymosins and Mucor miehei carboxyl protease were performed according to Towbin, et. al (35). Due to the high concentration of salt in chymosin culture broths and the effect this salt has on gel electrophoresis a desalting step was necessary. Pre-poured G-25 columns (Pharmacia, PD10) were equilibriated with 50 mM $Na_2HPO_4$ pH 6.0. A 2.5 ml aliquot of culture broth was applied to the column. The protein was eluted with 3.5 ml of the same buffer. The heterologous polypeptides present on the blots were detected by contacting the nitrocellulose blots first with rabbit anti-chymosin (36) or rabbit anti-Mucor miehei carboxy protease serum (36). The blots were next contacted with goat-anti-rabbit serum conjugated with horseradish peroxidase (Bio-Rad, Richmond, CA) and developed. Prior to loading on the gels, 50 μl of medium (desalted in the case of chymosin) was mixed with 25 μl of SDS sample buffer. β-mercaptoethanol was added to a final concetration of 1%. The sample was heated in a 95 ° C bath for 5 minutes after which 40-50 μl of sample was loaded on the gel. Each gel was also loaded with 2 μl each of 650, 65 and 6.5 μg/ml chymosin standards and molecular weight markers.

Western blots of pmeDJ1-7 transformants were similarly analyzed except that gel permeation was not performed.

Protease activity was detected as described by Sokol, et. al. (37). Luria broth was supplemented with 1-1.5% skim milk (Difco) and 30-35 ml was poured into a 150mm petri dish. An aliquot of 2 to 5 μl of culture medium was spotted on the plate. The plate was incubated over night at 37°C in a humidity box. The activity was determined based on the amount of milk clotting occurring on the plate measured in mm. The plates were co-spotted with dilutions of 100 CHU/ml or 16.6 CHU/ml rennin (CHU-Chr Hansen Unit, Chr Hansen's Laboratorium, A./S., Copenhagen). The relationship between the diameter of the coagulation zone (mm) and the centration of enzyme is logarithmic.

In order to distinguish between types of proteases, pepstatin, an inhibitor of the chymosin type of carboxyl protease, was used to inhibit protease activity attributable to chymosin. Samples of chymosin mutants and control broths were preincubated with a 1:100 dilution of 10mM pepstatin in DMSO for 5 minutes before analyzing for protease activity.

Glucoamylase secretion by pDJB-gam-1 transformants in 5% starch media was assessed using an assay based on the ability of glucoamylase to catalyze the conversion of p-nitrophenol-a-glucopyranoside (PNPAG) (38) to free glucose and p-nitrophenoxide. The substrate, PNPAG, was dissolved in DMSO at 150 mg/ml and 3 to 15 μl aliquots

were diluted to 200 ul with 0.2 M sodium acetate, 1mM calcium chloride at pH 4.3. A 25 µl sample was placed into a microtitre plate well. An equal volume of standards ranging from 0 to 10 Sigma A. niger units/ml (Sigma Chemical Co., St. Louis, MO) were placed in separate wells. To each well, 200 µl of PNPAG solution at 2.25 to 11.25 mg/ml was added. The reaction was allowed to proceed at 60 ° C for 0.5 to 1 hour. The time depended upon the concentration of enzyme. The reaction was terminated by the addition of 50 µl of 2 M trizma base. The plate was read at 405 nm. The concentration of enzyme was calculated from a standard curve.

Unless otherwise stated, chromosomal DNA was extracted from filamentous fungi by the following procedure. The filamentous fungus was grown in an appropriate medium broth for 3 to 4 days. Mycelia were harvested by filtering the culture through fine cheesecloth. The mycelia were rinsed thoroughly in a buffer of 50mM tris-HCl, pH7.5, 5mM EDTA. Excess liquid was removed by squeezing the mycelia in the cheesecloth. About 3 to 5 grams of wet mycelia were combined with an equivalent amount of sterile, acid-washed sand in a mortar and pestle. The mixture was ground for five minutes to form a fine paste. The mixture was ground for another five minutes after adding 10 ml of 50 mM tris-HCl, pH 7.5, 5mM EDTA. The slurry was poured into a 50 ml capped centrifuge tube and extracted with 25 ml of phenol-chloroform (equilibrated with an equal volume of 50 mM tris-HCl, pH 7.5. 5mM EDTA). The phases were separated by low speed centrifugation. The aqueous phase was saved and reextracted three times. The aqueous phases were combined (about 20 ml total volume) and mixed with 2 ml of 3 M sodium acetate, pH 5.4 in sterile centrifuge tubes. Ice cold isopropanol (25 ml) was added and the tubes were placed at -20°C for one hour. The tubes were then centrifuged at high speed to pellet the nucleic acids, and the supernatant fluid was discarded. Pellets were allowed to air dry for 30 minutes before resuspending in 400 µl of 10 mM tris-HCl, pH 7.5, 1mM EDTA (TE buffer). Pancreatic ribonuclease (Sigma Chemical Co., St. Louis, MO) was added to a final concentration of 10 µg per ml, and the tubes were incubated for 30 minutes at room temperature (30). Ribonuclease was then removed by extraction with phenol-chloroform. The aqueous layer was carefully removed and placed in a tube which contained 40 µl of 3M sodium acetate, pH 5.4. Ice cold ethanol was layered into the solution. The DNA precipitated at the interface and was spooled onto a glass rod. This DNA was dried and resuspended in a small volume (100 to 200 µl) of TE buffer. The concentration of DNA was determined spectrophotometrically at 260 nm (30).

To confirm the chromosomal integration of chymosin DNA sequences in selected transformants Southern hybridizations were performed (30). Spore suspensions of transformants were inoculated into Aspergillus complete medium and incubated at 37°C on a rotary shaker for 24-48 hrs. The medium was non-selective in that it was supplemented with 5 ppm p-aminobenzoic acid and contained sufficient uracil for growth of the auxotrophic parent. In effect, these Southerns also tested, for the stability of the transformants. The mycelium was filtered, ground in sand, and the DNA purified as previously described. Transformant DNA was then digested with various restriction enzymes and fragments separated by agarose gel electrophoresis. Control lanes included digested pDJB3 transformant DNA and undigested DNA. Gels were stained with ethidium bromide, photographed, blotted to nitrocellulose or nytran (Schleicher and Schuell, Keene, NH), and probed with radiolabeled plasmids or specific fragments.

Some of the following examples illustrate the invention as claimed; others provide useful background.

EXAMPLE 1

Expression and Secretion of Aspergillus niger glucoamylase By Aspergillus Nidulans

A. Construction of pGA1

Aspergillus niger (Culture #7, Culture Collection Genencor, Inc., South San Francisco, CA.) was grown in potato dextrose broth (Difco, Detroit, MI) at 30°C for 3 days with vigorous aeration. Chromosomal DNA was extracted as previously described.

A synthetic oligonudeotide was used as a hybridization probe to detect the glucoamylase gene from Aspergillus niger. The oligonucleotide was 28 bases in length (28mer) and corresponded to the first 9 1/3 codons of the published glucoamylase coding sequence (39):

MetSerPheArgSerLeuLeuAlaLeuSer
5'ATGTCGTTCCGATCTCTACTCGCCCTGA 3'

The oligonucleotide was synthesized on a Biosearch automated DNA synthesizer (Biosearch, San Rafael, CA) using the reagents and protocols specified by the manufacturer.

Genomic DNA from Aspergillus niger was analyzed for the presence of glucoamylase sequences by the method of Southern (30). Briefly, 10 µg of Aspergillus niger DNA was digested with EcoRI restriction endonuclease. The digested DNA was subjected to electrophoresis on a 1% agarose gel according to standard methods (30). DNA was transferred from the gel to a nitrocellulose membrane (Schleicher & Schuell, Inc., Keene, NH) by blotting In 10x SSC (1.5 M NaCl, 0.15 M trisodium citrate) (30). DNA was fixed to the nitrocellulose by baking in an 80°C vacuum oven, followed by hybridization at low stringency (2,40) with radiolabeled oligonucleotide probe. Radiolabeling of the synthetic oligonucleotide was done at 37°C In a 50µl reaction that contained 70 mM tris-HCl. pH 7.5, 10 mM $MgCl_2$, 5 mM dithiothreitol, 30 pmoles of synthetic oligonucleotide, 20 pmoles of gamma-[32P]ATP (Amersham, Chicago, Il; specific activity 5000 Ci/mmol), and 5 units of T4 polynucleotide kinase (New England Biolabs). After hybridization, the filters were washed 15 minutes in 2xSSC, 0.1% sodium dodecylsulfate (SDS) and twice in 2xSSC at 37°C. Filters were air dried, wrapped in Saran-Wrap (Dow Chemical) and applied to Kodak XOmat-AR X-ray film at -70°C to obtain an autoradiographic image. After developing the autoradiogram, a band of hybridization was clearly visible corresponding to a 3.5 kilobase-pair EcoRI fragment.

Genomic DNA from Aspergillus niger was digested with EcoRI and size-fractionated by polyacrylamide gel electrophoresis according to standard methods (30). DNA fragments 3 to 4 kb in size were excised and eluted from the gel (30). This DNA fraction was used to generate a library of clones in the Escherichia coli cloning vector pBR322 (ATCC 37017). The cloning vector was cleaved with EcoRI and dephosphorylated with bacterial alkaline phosphatase (Bethesda Research Labs). A typical dephosphorylation reaction consisted of 1µg of digested vector DNA and 1 unit of alkaline phosphatase in 50 µl of 50 mM tris-HCl, pH 8.0, 50 mM Nacl. The reaction was incubated at 65° C for one hour. The phosphatase was removed by extraction with phenol-chloroform. The EcoRI, size-selected Aspergillus niger DNA was then ligated with EcoRI cleaved and dephosphorylated pBR322. A typical ligation reaction contained the following: 100 ng each of vector and insert DNAs, 1 unit of T4 DNA ligase (Bethesda Research Labs), 25 mM tris-HCl, pH 7.5, 10 mM $MgCl_2$, 10 mM dithiothreitol, and 1 mM ATP in a 10µl volume. Ligation reactions were incubated at 16° C for 18 to 24 hours. The ligated DNA was used to transform competent E. coli. 294 cells (ATCC 31446) prepared by the method of Morrison (41). Transformants were selected on LB agar plates (30) which contained carbenecillin at a final concentration of 50 µg per ml. Transformants which harbored glucoamylase gene sequences were identified by colony hybridization methods (30) using the glucoamylase-specific 28 mer as a probe. Hybridizing colonies were purified, and plasmid DNAs were isolated from each by the alkaline-SDS miniscreen procedure (30). The plasmids selected in this manner all contained a 3.5 kb EcoRI fragment which hybridized to the synthetic glucoamylase probe. One such plasmid, designated pGa1, was selected for further analysis. A 1.1 kb EcoRI-BglII fragment from the insert in pGal was subcloned into M13 mp9 (42) and partially sequenced by the dideoxy chain termination method (43) to confirm that the cloned DNA encoded the glucoamylase gene. A restriction endonuclease cleavage map of the 3.5 kb EcoRI fragment contained in pGal is depicted in Figure 1. It was generated by single and double restriction digests followed by orientation of the DNA fragments with respect to known restriction sites in pBR322 (44).

B. Construction of pGa5

The nucleotide sequence and restriction map of pGa1 indicated that pGa1 contained the entire glucoamylase coding region and 221 nucleotides of 5' flanking DNA. The sequences in this 5' region were strikingly similar to typical eukaryotic promoter sequences with TATAAAT and CAAT boxes located upstream of the ATG start codon (48).

However, to insure that possible upstream activation sites of the Aspergillus niger glucoamylase gene were included in the final transformation vector a larger genomic fragment which contained at least 1000 bp of 5' flanking DNA was cloned. Southern blotting experiments similar to those already described identified a 6.5 kb ClaI fragment which hybridized to a radiolabeled EcoRI glucoamylase fragment from pGa1. The EcoRI fragment was radiolabeled by nick translation (30) with alpha-[32P]dCTP (Amersham; specific activity 3000 Ci/mmol). A nick translation kit (Bethesda Research Labs) was used for the labeling reaction by following the instructions supplied by the manufacturer. Filters were hybridized and washed under stringent conditions (30).

The 6.5 kb ClaI fragment identified by hybridization was cloned in a manner similar to that described previously. Aspergillus niger DNA was digested with ClaI and size-fractionated by polyacrylamide gel electrophoresis. DNA fragments migrating between 5.5 and 8 kb were excised and eluted from the gel. This fraction was ligated to ClaI cleaved and dephosporylated pBR325 (45). The ligation mixture was used to transform competent E. coli 294 cells. Transformants were selected on LB agar plates containing carbenecillin (50 µg/ml). Colonies which contained glucoamylase gene sequences were identified by colony hybridization (30). Plasmid DNA extracted from hybridizing colonies contained a 6.5 kb ClaI fragment which included the 3.5 kb EcoRI fragment cloned previously in pGa1. These recombinant plasmids encoded the Aspergillus niger glucoamylase gene as confirmed by supercoil-DNA sequencing (46) with the synthetic oligonucleotide (28 mer) as a sequencing primer. A restriction endonuclease cleavage map of the 6.5 kb ClaI fragment was constructed using single and double digests of the DNA cloned in pBR325. Restriction sites in the vector DNA were used as reference points to orient the fragment. This restriction map is shown in Figure 1. Location of the glucoamylase gene was deduced by comparing restriction sites of pGa5 to those of the previously published glucoamylase genes (39, 47, 48). From the mapping data it was estimated that approximately 3.3 kb of the 5'-flanking DNA and about 1 kb of 3'-flanking DNA were contained within the cloned fragment.

Plasmid pGa5 was deposited with the ATCC on August 28, 1985 in E. coli 294 and has been assigned number 53249.

C. Vector for Expression and Secretion of Aspergillus niger Glucoamylase

The 6.5 kb ClaI fragment from pGa5 containing the glucoamylase gene was cloned into the E. coli.-Aspergillus nidulans shuttle vector pDJB3 as depicted in Figure 2. The pDJB3 shuttle vector possesses a selectable beta-lactamase gene and origin or replication from E. coli plasmid pBR325, the pyr4 gene from Neuospora crassa which relieves the auxotrophic requirement for uridine in Aspergillus nidulans strain G191, a sequence known as ANS1 from Aspergillus nidulans which promotes a high frequency of stable integrative transformants in Aspergillus nidulans, unique EcoRI and ClaI restriction sites for cloning.

pDJB is constructed as depicted in Figure 14. Plasmid pFB6 (32) is digested to completion with BglII and partially digested with HindIII. Fragment B containing the pyr4 gene (ca. 2Kb) is purified by gel electrophoresis and ligated into HindIII/Bam HI digested pBR325 (fragment A) yielding plasmid pDJB1. The ANS-1 sequence is cloned by ligating EcoRI digested A. nidulans genomic DNA (strain G191 or other FGSC#4- derived strains) into EcoRI cleaved pFB6. The resulting pool of EcoRI fragments in pFB6 is used to transform a ura3- S. cerevisiae (E.G. ATCC 44769, 44770 etc.). An autonomously replicating plasmid, pIntA, is purified from the S. cerevisiae transformant. pIntA is digested with EcoRI, the ANS-1 fragment is purified by gel electrophoresis and ligated into EcoRI digested pDJB1, yielding plasmid pDJB2. pDJB2 is partially digested with EcoRI, treated with DNA polymerase I (Klenow), and re-ligated to yield plasmid pDJB3. The partial nucleotide sequence and restriction map of the ANS-1 fragment is showin in Figure 13A and 13B.

Plasmid pGa5 was digested with ClaI and the large fragment (fragment A) was separated from the vector by agarose gel electrophoresis. This fragment was ligated with pDJB3 which had been cleaved with ClaI and dephosphorylated (fragment B). The ligation mixture was used to transform competent E. coli 294 cells. Transformants were selected on LB agar supplemented with carbenecillin (50 µg/ml). Analysis of plasmid DNAs from these transformants indicated that the glucoamylase fragment had been inserted as expected. Both orientations of the glucoamylase fragment were obtained by screening various transformants. One plasmid, designated pDJB-gam1 was arbitrarily chosen for transformation of Aspergillus nidulans protoplasts.

D. Expression and Secretion of Glucoamylase

Aspergillus nidulans Strain G191 was transformed with pDJB-gam-1 as previously described. Five transformants designated pDJB-gam-1-4, 9, 10, 11 & 13 were analyzed for glucoamylase activity as previously described. The results are shown in Table I.

Table I

| Sample | Glucoamylase Activity (Sigma Units/ml) |
|---|---|
| pDJB3 | 0.129 |
| pDJB-gam-1-4 | 0.684 |
| pDJB-gam-1-9 | 0.662 |
| pDJB-gam-1-10 | 0.131 |
| pDJB-gam-1-11 | 0.509 |
| pDJB-gam-1-13 | 0.565 |
| A. niger | 2.698 |

As can be seen, each pDJB-gam-1 transformant produced more glucoamylase activity than the control indicating that biologically active glucoamylase was expressed and secreted from the transformed fungi.

EXAMPLE 2

Expression and Secretion of Bovine Chymosin from Aspergillus nidulans

Expression vectors were constructed encoding either a natural precursor of bovine chymosin (preprochymosin) or a fusion precursor in which DNA sequences for Aspergillus niger glucoamylase and prochymosin were precisely fused.The strategy for the construction of these vectors involved the following steps. First, a DNA sequence containing a portion of the glucoamylase promoter and a portion of the glucoamylase 5'-coding region was cloned upstream from a DNA sequence corresponding to the amino-terminal portion of preprochymosin. Next, nucleotides between the DNA fragments were deleted by M13 site-specific mutagenesis (40) using specific primer sequences. Finally, a segment of DNA containing the fused sequences was incorporated with the remaining portion of the prochymosin sequence into an expression vector which employed the 5'- and 3'- regulatory sequences of the Aspergillus niger glucoamylase gene. These steps are outlined in Figures 3 through 7.

A. Construction of mp19 GAPR

Plasmid pGa5 is used to derive a 337 bp EcoRI-RsaI DNA fragment (fragment A) bearing a portion of the glucoamylase promoter and an amino-terminal segment of the coding region. Fragment A was ligated with EcoRI and SmaI digested M13mp19 RF-DNA (fragment B). The ligation mixture was used to transform E. coli. JM101 (ATCC 33876). Clear plaques were analyzed for the presence of fragment A by restriction analysis of the corresponding RF-DNA. One isolate containing fragment A, designated mp19R-Rsa was digested with PstI and XbaI and the large fragment (fragment C) was isolated. A small XbaI-PstI sequence (fragment D) derived from pR3 (49) containing 5' preprochymosin sequences; was purified by electrophoresis and ligated to fragment C to produce the phage template mp19GAPR as shown in Figure 3.

B. Site Specific Deletion Mutagenesis

As shown in Figure 8 mp19GAPRΔC1 was derived from mp19 GAPR by deleting the nucleotides between the glucoamylase signal peptide codons and the codons for prochymosin by site-specific mutagenesis. Thus, in mp19GAPRΔC1 the glucoamylase signal peptide codons are precisely fused to the first codon of prochymosin. Site-specific mutagenesis was done as previously described (40) except that only one oligonucleotide was used to prime second strand synthesis on the single-stranded M13 template (Figure 4) (40). The synthetic oligonucleotide used to derive mp19GAPRΔC1 (primer 1) was 5' GCTCGGGGTTGGCAGCTGAGATCACCAG 3'. Plaques containing the desired deletion were identified by hybridization with the primer radio-labeled as previously described.

In mp19GAPRΔC3 the nucleotides between those immediately preceding the initiation codon of glucoamylase and the ATG start codon of preprochymosin were joined by site-specific mutagenesis using the synthetic oligonucleotide (primer 3)

5′ ACTCCCCCACCGCAATGAGGTGTCTCGT 3′.

The resulting mutation linked the glucoamylase promoter region precisely to the initiation codon of preprochymosin as depicted in Figure 8.

C. Construction of Vectors for the Expression and Secretion of Bovine Chymosin

As further depicted in Figure 4 each of the fusions between the glucoamylase sequences and 5' prochymosin sequences (m19GAPRΔC1 and mp19GAPRΔC3) were combined with the 3' prochymosin sequences and the Saccharomyces cerevisiae phosphoglyceratekinase (PGK) terminator in the Aspergillus nidulans transformation vector pDJB3. The replicative form of mp19GAPRΔC1 and mp19GAPRΔC3 was digested with EcoRI and PstI. The smaller fragment (fragment 1) was isolated. Plasmid pBR322 was also digested with EcoRI and PstI and the larger vector fragment (fragment 2) was isolated. Fragments 1 and 2 were joined by ligation and used to transform E. coli. 294. A tetracycline resistant colony containing either plasmid pBR322GAPRΔC1, or pBR322GAPRΔC3 was isolated. Fragment 2 was also treated with E. coli. polymerase I (Klenow fragment). The resulting blunt ended fragment was circularized by ligation and used to transform E. coli 294. One tetracycline resistant colony containing plasmid pBR322ΔRP was isolated and then digested with HindIII and SalI. The larger vector fragment (fragment 3) was isolated. The plasmid pCR160 was digested with HindIII and PstI and fragment 5 (containing the yeast PGK terminator fused to 3' prochymosin codons) was isolated. Fragments 3, 4, and 5 were joined by ligation and used to transform E. coli 294. A tetracycline resistant transformant containing plasmid pBR322GAPR$^{\Delta}$C1 or pBR322GAPR$^{\Delta}$C3 was isolated.

Plasmid pCR160 contains the yeast 2 μm origin of replication to allow its maintenance as a plasmid in yeast, the yeast TRP-1 gene as a yeast selection marker, an E. coli origin or replication and ampicillin resistance gene from the plasmid pBR322, and a prorennin expression unit. The prorennin expression unit contained the promoter from the yeast PGK gene, the prorennin coding region, and the terminator from the PGK gene. Construction of this plasmid was accomplished as depicted in Figure 9 in the following manner: Plasmid YEpIPT (50) was partially digested with HindIII followed by a complete EcoRI digestion, and the vector fragment A was isolated. A second plasmid pPGK-1600 (51) was partially digested with both EcoRI and HindIII, and the PGK promoter fragment B was isolated. Fragments A and B were ligated to give the intermediate pIntI which was again partially digested with EcoRI and the HindIII, and the vector fragment C was isolated. The PGK terminator fragment D was isolated following HindIII and Sau3A digestion of the plasmid pBI (52). The prorennin fragment E was isolated by cleaving pRI (49) DNA with EcoRI and BclI. Fragments C, D, and E were then ligated to produce the yeast expression plasmid pCR160. The nucleotide sequence of the PGK promoter, structural gene and terminator have been reported (53).

Plasmids pBR322GAPRΔC1 and pBR322GAPRΔC3 contain a complete transcriptional unit for each of the forms of prochymosin. This transcriptional unit contains a precursor prochymosin coding sequence, the glucoamylase promoter, and the yeast PGK terminator. However, derivatives of these plasmids and plasmids pBR322GAPRΔC2 and pBR322GAPRΔ4, described hereinafter, [designated pIntI (1-4) Figure 5)] produced no detectable chymosin when used to transform A. nidulans G191. It is not understood why these derivative plasmids failed to express and secrete chymosin. However, in light of subsequent results it appears that the yeast PKG terminator and/or the short glucoamylase promotor sequence in these plasmids is not recognized by A. nidulans G191. Based on these results, the pBR322GAPRΔC plasmids were further modified.

In the following steps the transcriptional unit was moved onto the Aspergillus nidulans transformation vector pDJB3. Additional glucoamylase 5' flanking sequences were incorporated just 5' of the promoter to insure the presence of possible upstream activation sites which could be involved in regulating expression. Further, the PKG terminator was replaced with the A. niger glucoamylase terminator from pGa5. Specifically, in Figure 5 each plasmid (pBR322GAPRΔC1 or pBR322GAPRΔC2) was digested with ClaI and fragment 6 was isolated. Plasmid pDJB3 was also digested with ClaI and treated with bacterial alkaline phosphatase in order to minimize self-ligation. This digested plasmid (fragment 7) was joined to fragment 6 and one ampicillin resistant colony containing plasmid pINTI-1 or pIntI-3 was isolated. These plasmids were digested with XhoI and NsiI and the larger vector fragment (fragment 8 ) was isolated. Plasmid pGa5 which contains the entire glucoamylase gene as well as extensive 5' and 3' flanking sequences was digested with XhoI and NsiI and the smaller fragment (fragment 9, containing approximately 1700 bp of these 5' sequences) was isolated. Fragments 8 and 9 were joined by ligation and used to transform E. coli 294. One ampicillin resistant colony containing plasmid pInt2-1 or pInt2-3 was isolated. These plasmids differ most significantly from the final vectors (see Figure 7) in that they contain the yeast PGK terminator rather than the glucoamylase terminator.

Additional steps in the construction of chymosin expression vectors are outlined in Figure 6. Plasmid pRI (49) was used to isolate a small BclI-Asp718 DNA fragment (fragment A) which comprised the 3'- end of prochymosin cDNA. Fragment A was subsequently cloned into pUC18 (33) that was digested with Asp718 and BamHI (fragment B). Similarly, a 1.2 kb ClaI-Asp718 DNA fragment (fragment D) was isolated from plasmid pGa5, and cloned into AccI and Asp718 cleaved pUC18 (fragment C). The resulting intermediate plasmids, pUC-intl and pUC-int2, were digested with SalI and HindIII, and fragments E and F were isolated. These fragments were then ligated to produce pUC-int3 which contained the 3' end of prochymosin followed by the glucoamylase terminator sequences on a HindIII-Asp718 fragment (fragment H).

A new cloning vector, designated pBR-link, was created by inserting a synthetic oligonucleotide linker (containing XhoI and ClaI sites) into the unique BamHI site of pBR322. This linker connoted the following sequence:

```
5' GATCCATCGATCTCGAGATCGATC 3'
       3' GTAGCTAGAGCTCTAGCTACCTAG 5'
```

The larger HindIII-XhoI fragment of this vector (fragment G) was purified by electrophoresis. Similarly, the XhoI-Asp718 restriction fragments (fragments I) of plasmids pInt2-1 and pInt2-3 were isolated electrophoretically. Fragments G and H were ligated with each of the different I-fragments in a series of three-way ligations to produce the intermediates pInt3-1 and pInt3-3. These key intermediates contained the glucoamylase promoter regions, various signal and propeptide fusions to the prochymosin (or preprochymosin) sequences followed by the glucoamylase terminator region all within convenient ClaI restriction sites. Because certain ClaI sites, such as those in the linker of pBR-link, are inhibited by E. coli. DNA methylation, the plasmids pInt3-1 through pInt3-4 were transformed into a dam-strain of E. coli, designated GM48, (ATCC 39099) from which the plasmids were re-isolated. The unmethylated DNA was digested with ClaI and fragment J was purified by electrophoresis. Fragment J from each of the glucoamylase-prochymosin fusions was subsequently cloned into the unique ClaI site of pDJB3 (fragment K) to produce the final expression vectors pGRG1 and pGRG3.

D. Expression and Secretion of Bovine Chymosin

Aspergillus nidulans G191 was transformed with pGRG1 and pGRG3 as previously described.

Five pGRG1 and five pGRG3 transformants were analyzed. Western analysis (not shown) indicated that each transformant secreted a protein which reacted with anti-chymosin and which migrated at the same or slightly higher molecular weight of bovine chymosin. The higher molecular weight species may be due to incorrect processing, media effects, or glycosylation. Integration was confirmed for one transformant of pGRG3 by Southern hybridization (results not shown). Each transformant was also assayed for chymosin activity. The results of this assay are shown in Table II.

Table II

| Transformant | No. of Transformants Tested | Range of Chymosin Activity μg/ml |
|---|---|---|
| pDJB3 | 1 | 0-0.13 |
| pGRG1 | 5 | 0- 1.5 |
| pGRG3 | 5 | 0.05 - 7.0 |

These results indicate that pGRG1 and pGRG3 both secrete a protease, at various levels, above the pDJB3 control. Occasionally, background proteolytic activity was detected in pDJB3 control broths. As will be shown hereinafter this protease activity of transformants is associated with the aspartic acid family of carboxyl proteases of which chymosin is a member.

EXAMPLE 3

Expression and Secretion of Fusion Polypeptides from Aspergillus Nidulans

Two fusion polypeptides were constructed for expression and secretion from A. nidulans. One fusion polypeptide contained an amino-terminal portion consisting of the pro sequence and first ten amino acids of Aspergillus niger glucoamylase and a carboxyl-terminal portion consisting of bovine prochymosin. The second fusion polypeptide contained an amino-terminal portion consisting of the pro sequence only of Aspergillus niger glucoamylase and a carboxyl-terminal portion consisting of bovine prochymosin.

A. Vectors for Expressing and Secreting Fusion Polypeptides

Vectors encoding the above fusion polypeptides were constructed by deleting specific sequences from mp19GAPR followed by the same manipulations as described above for constructing pGRG1 and pGRG3. As shown in Figure 8, in mp19GAPRΔC2 the nucleotides between the glucoamylase propeptide codons and the codons of prochymosin were deleted using the site-specific mutagenesis method described above. The sequence of the oligonucleotide synthesized for this mutagenesis (primer 2) was

5′ TGATTTCCAAGCGCGCTGAGATCACCAG 3′.

This mutation was intended to fuse the glucoamylase promoter, signal peptide, and propeptide codons to the first codon of prochymosin. In mp19GAPRΔC4 the nucleotide sequences between the tenth codon of mature glucoamylase and the codons of prochymosin were deleted by M13 site-specific mutagenesis with the synthetic oligonucleotide (primer 4)

5′ TGAGCAACGAAGCGGCTGAGATCACCAG 3′.

This deletion fused the glucoamylase promoter region, signal peptide sequence, propeptide sequence, plus ten codons of the mature glucoamylase to the codons of prochymosin as shown in Figure 8. These expression and secretion vectors designated as pGRG2 and pGRG4 were used to transform A. nidulans.

B. Expression and Secretion of Chymosin from Aspergillus Nidulans Transformed With PGRG2 and PGRG4

pGRG2 and pGRG4 transformants were cultured as previously described. The culture medium was assayed for chymosin activity by Western blot and gave results similar to those obtained for pGRG1 and pGRG3. Integration of one pGRG2 transformant was confirmed by Southern Analysis (results not shown). The results of the chymosin assay are presented in Table III.

Table III

| Transformant | No. of Transformants Tested | Range of Chymosin Activity μg/ml |
|---|---|---|
| pDJB3 | 1 | 0-0.13 |
| pGRG2 | 1 | 0.001-0.42 |
| pGRG4 | 6 | 0.004-0.75 |

Again each of the transformants demonstrated protease activity above the pDJB3 control indicating that a protease was expressed and secreted by the transformants. As with pGRG1 and pGRG3, these proteases belong to the aspartic acid family of carboxyl proteases as evidenced by the pepstatin inhibition. Significantly, these results indicate that hybrid polypeptides have been expressed in a filamentous fungus.

EXAMPLE 4

Pepstatin Inhibition Study

Three of the above vectors containing the various constructions involving chymosin were analyzed in the pepstatin inhibition assay as described supra. The results are shown in Table IV.

Table IV

| Sample | Chymosin Activity (μg/ml) |
|---|---|
| pDJB3 | 0 |
| pDJB3 pepstatin | 0 |
| pGRG1 | 0.2 |
| pGRG1 pepstatin | 0.05 |
| pGRG2 | 0.1 |
| pGRG2 pepstatin | 0 |
| pGRG3 | 3 |
| pGRG3 pepstatin | 0.6 |

The samples preincubated with pepstatin show a marked decrease in activity indicating that the protease produced by the transformants is of the aspartic acid family of acid proteases to whic chymosin is a member. This data together with the results from the Western analysis indicates that biologically active chymosin is expressed and secreted by A. nidulans G191 transformed with pGRG1, pGRG2, pGRG3 and pGRG4.

The variation in the amount of chymosin activity detected for different vector constructions in Example II and Example III may reflect differences in the recognition of the various signals incorporated in each transformation vector. Within a particular construction, the variation in chymosin activity may be related to the copy number of the vector incorporated into the fungal genome and/or to the location of such integration.

EXAMPLE 5

Carbon source studies

One vector, pGRG4, was used to transform A. nidulans G191 which was thereafter grown on the various carbon sources previously described. The results of this assay are shown in Table V.

Table V

| Amount of chymosin activity produced on various carbon sources (μg/ml) | | | | |
|---|---|---|---|---|
| | starch | glucose | fructose | sucrose |
| pDJB3 | 0 | 0 | 0 | 0 |
| pGRG4 | 3.5 | 3.5 | 0.9 | 1.75 |

These results clearly show that chymosin is secreted regardless of the carbon source. This suggests that transcriptional regulation of the glucoamylase promotor is unlike that in A. niger, i.e. not strongly inducible by starch.

EXAMPLE 6

Expression and Secretion of Mucor meihei Carboxyl Protease

A. Carboxyl Protease Genomic Probe

The partial primary structure of Mucor miehei acid protease (54) was inspected for the region of lowest genetic redundancy. Residues 187-191 (using the pig pepsin numbering system), try-tyr-phe-trp-asp, were selected. Oligonucleotides complementary to the coding sequence corresponding to this amino acid sequence,

5′-GC(G/A)TCCCA(G/A)AA(G/A)TA(G/A)TA-3′,

were synthesized (31) and labelled using gamma 32P-ATP and T4 polynucleotide kinase (30).

B. Cloning of Mucor meihei Carboxyl Protease

Genomic DNA from Mucor miehei (Centraal Bureau Voor Schimmelcultures, Holland 370.75) was prepared as follows. Cells grown in YMB medium (3g/l yeast extract, 3g/l malt extract, 5g/l peptone, 10g/l glucose) were collected by centrifugation, washed twice with 0.5M NaCl, and lyophilized. Cell walls were then disrupted by adding sand to the cells and grinding the mixture with a mortar and pestle. The resulting powder was suspended (15 ml. per gram dry weight) in a solution containing 25% sucrose, 50mM Tris-HCl (pH 8.0), and 10 mM EDTA. SDS was added to a final concentration of 0.1% and the suspension was extracted once with a half-volume of phenol and three times with half volumes of chloroform. The final aqueous phase was dialysed extensively against 10mM Tris-HCl, pH 8.0 and 1mM EDTA. The DNA was then precipitated by the addition of sodium acetate, pH 5.5, to a concentration of 0.3 M. followed by the addition of 2.5 volumes of cold ethanol. Aliquots of this DNA were digested with a variety of restriction endonucleases according to the manufacturers' directions and then analyzed for sequences complementary to sequences of the probes described above, using the method of Southern. A positively hybridizing band of approximately 2.5 kb (kilobases) was identified in the HindIII digested DNA. HindIII digested genomic DNA was separated by polyacrylamide gel electrophoresis and a gel fragment containing DNA of 2.0-3.0 kb was electroeluted as previously described. The electroeluted DNA, presumed to be enriched for sequences corresponding to the Mucor miehei acid protease gene, was ethanol precipitated. The cloning vector pBR322 (ATCC 37017) was digested with HindIII and dephosphorylated using bacterial alkaline phosphatase. In a typical 10 ul reaction 100 ng of vector and 100 mg of the size enriched DNA were joined in the presence of ATP and T4 DNA ligase. The reaction was used to transform E. coli 294 (ATCC 31446) by the calcium shock procedure (30). About $2.0 \times 10^4$ ampicillin resistant clones were obtained. Approximately 98% of these contained cloned inserts as indicated by their failure to grown on tetracycline containing medium. These colonies were tested by a standard colony hybridization procedure for the presence of sequences complementary to those of the DNA probes. One positively hybridizing colony, containing plasmid pMe5'muc, was found to contain a HindIII insert of the expected 2.5 kb size. The termini of this fragment were subcloned into M13 sequencing vectors (33) and their sequences determined by the dideoxy chain termination method. One terminus contained sequences corresponding to the known amino terminal amino acid sequence of the acid protease gene. The adjacent 3' region was sequenced in order to obtain more C terminal coding sequences. The sequencing strategy is shown in Fig. 10. In this way the entire coding sequence for the mature form of the protein was obtained. The 5' end of the fragment was found to occur 112 bp (base pairs) upstream of the codon corresponding the mature amino terminus. Since this upstream region contained no in frame initiation codons it was presumed to be part of a propeptide.

In order to obtain DNA containing the initiation codon as well as 5' untranslated sequences a more 5' clone was isolated as follows. A HindIII-ClaI 813 bP 5' subfragment of the pMe5'Cla Hind III insert was isolated and labelled by the nick translation method (30). This labelled fragment was used to probe ClaI digested Mucor miehei genomic DNA by the method of Southern. This experiment revealed a single band of hybridization corresponding to a molecular weight of approximately 1300 bp. Size enriched DNA of this size was isolated and cloned into ClaI digested and de-phosphorylated pBR322 as described above.

Approximately 9000 ampicillin resistant colonies were obtained. About 90% of these contained cloned inserts as indicated by their failure to grow on tetracycline containing medium. These colonies were tested by a standard colony hybridization procedure for the presence of sequences complementary to those of the nick translated probe. One positively hybridizing colony, containing plasmid pMe2, was found to contain a ClaI insert of the expected 1.3 kb size. Sequencing of the ends of this fragment showed that one terminus corresponded to sequences near the ClaI site of the Hind III fragment in pMe5'Cla and thus permitted orientation of the fragment which is shown in Figure 10. Further sequencing of the ClaI fragment disclosed the initiation codon and 5' untranslated sequences. The entire coding sequence and the 5' and 3' flanking sequences are shown in Figure 10. Comparison of the deduced primary structure with that determined by direct amino acid sequencing indicates that the Mucor protein is made as a precursor with an amino-terminal extension of 69 residues. Based on the structural features generally present in leader peptides it is likely that residues -21 to -1 comprise a leader peptide and that residues 21-69 comprise a propeptide analogous to that found in the zymogen forms of other acid proteases including chymosin and pepsin (55).

C. Mucor meihei carboxyl protease Expression and Secretion Vector

A vector for expressing and secreting Mucor miehei carboxy protease includes the entire native Mucor miehei acid protease transcriptional unit including the coding sequence, 5' flanking sequences (promoter), and 3' flanking sequences (terminator and polyadenylation site).

The overall strategy for making this vector is depicted in Fig. 12. The Aspergillus nidulans transformation vector pDJB1 was digested with ClaI and EcoRI and the larger vector fragment (fragment 1) was isolated. The plasmid pMe5'Cla was digested with EcoRI and ClaI and fragment 2 was isolated. This fragment contains the 5' codons of the acid protease together with about 500 bp of 5' flanking sequences. Fragments 1 and 2 were joined by ligation and used to transform E. coli 294. One ampicillin resistant colony containing plasmid pMeJBint was isolated. This plasmid was digested with ClaI and treated with bacterial alkaline phosphatase in order to reduce self ligation and is designated fragment 3. Plasmid pMe2 was digested with ClaI and the smaller fragment (fragment 4) was isolated. This fragment contains the Mucor miehei acid protease 3' codons and about 1800 bp of 3' flanking sequences. Fragments 3 and 4 were joined by ligation and used to transform E. coli 294. One ampicillin resistant colony containing plasmid pMeJB1-7 was isolated. This vector was used to transform Aspergillus nidulans.

D. Expression and Secretion of Mucor miehei Carboxyl Protease by Aspergillus Nidulans

Southern blot analysis of six transformants indicated the presence of the entire Mucor miehei acid protease gene in the Aspergillus nidulans genome (results not shown). In addition, each of the transformants were analyzed by Western blots (results not shown) and for acid protease activity. The results of the protease assay are shown in Table VI.

Table VI

| Transformant | Protease Activity (mg/ml) |
|---|---|
| 1 | 0.003 |
| 2 | 0.007 |
| 3 | 0.003 |
| 4 | 0.005 |
| 5 | 0.005 |
| 6 | 0.012 |

These experiments demonstrate expression and secretion of a protein that reacts with specific antibody to Mucor miehei carboxyl protease and which has milk clotting activity. The protein has an apparent molecular weight by electrophoretic analysis that is slightly greater than that of the authentic (Mucor miehei derived) protein. This may indicate that Aspergillus nidulans glycosylates this glycoprotein to a different extent than Mucor miehei. Because the Aspergillus nidulans derived Mucor meihei acid protease appears to have the same specific activity as the authentic material it appears that it has been processed (by the cell or autocatalytically) to the mature form. The unprocessed forms of other acid proteases such as chymosin and pepsin are zymogens which require processing (autocatalytic) before activity is obtained.

The varying levels of expression in the various transformants may reflect the position or copy number of the protease gene in the Aspergillus nidulans genome. However, the expression and secretion of biologically active carboxyl protease indicates the A. nidulans recognizes the promoter, signal and terminator signals of Mucor miehei carboxyl protease.

EXAMPLE 7

**Expression and Secretion of Chymosin Encoded by pGRG1-4 from A. Awamori and Trichoderma reesei pyrG Auxotrophic Mutants**

The plasmids pGRG1 through pGRG4 (pGRG1-4) were also used to transform orotidine-5'-phosphate decarboxylase (OMPCase) deficient mutants of A. awamori and Trichoderma reesei. The pyr4 gene from N. crassa encoded by the pGRG1-4 plasmid complements these OMPCase mutants in the absence of uridine to permit the isolation of successful transformants. The thus transformed mutants of A. awamori and T. reesei secreted detectable amounts of bichemically active chymosin into the culture medium.

A. Production of pyrG Auxotrophs

The method used to obtain pyrG auxotrophic mutants of A. awamori and T. reesei involved selection on the pyrimidine analog 5-fluoro-orotic acid (FOA) (56). The mechanism by which FOA kills wild-type cells is unknown. However, in view of the resistance of OMPCase-deficient mutants to FOA, it is likely that the toxicity occurs through conversion of FOA to 5-fluoro-UMP. Whether cell death is caused by a flouoridated ribonucleotide or deoxyribonucleotide is uncertain.

The following methods describe the isolation of OMPCase-deficient (FOA-resistant) mutants of T. reesei and A. awamori:

1. Trichoderma reesei

A fresh spore suspension of T. reesei strain P37 (NRRL 15709) was washed three times in sterile distilled water containing 0.01% Tween-80. Fifteen milliliters of this spore suspension ($1 \times 10^7$ spores per ml) were placed in a sterile petri dish ($100 \times 20$ mm) with a sterile magnetic stirring bar. The lid was removed and the spores were exposed to ultraviolet (UV) light at 254 nm (7000 uW per cm2), in the dark at a distance of 25 cm from the UV light source. The spores were stirred constantly. UV exposure continued for three minutes (sufficient to give 70% killed spores). The irradiated spore suspension was collected in a 50 ml centrifuge tube and stored in the dark for one hour to prevent photoreactivation. Spores were pelleted by centrifugation and the pellet was resuspended in 200 ul of sterile water containing 0.01% Tween-80.

The suspension was diluted and plated onto YNB agar medium (0.7% yeast nitrogen base without amino acids, 2% glucose, 10mM uridine, 2% agar) (56) containing 0.15% FOA (SCM Specialty Chemicals, Gainsville, Florida). After 4 days incubation at 30°C, 75 colonies were picked to fresh YNB agar that contained FOA. Sixty-two of the 75 colonies grew and were toothpicked to minimal agar (6 g/l $NaNO_3$, 0.52 g/l KCl, 1.52 g/l $KH_2PO_4$, 1 ml/l trace elements solution, 1% glucose, 0.1% $MgSO_4$, 20 g/l agar) and minimal agar plus 1 mg/ml uridine to determine uridine requirements. All of the 62 isolates grew on minimal agar with uridine, but 9 isolates failed to grow on minimal agar alone. These 9 strains were repicked to minimal agar and minimal agar with uridine. Two of the strains grew only on minimal agar supplemented with uridine. One of these, designated T. reesei pyrG29, grew well on minimal medium with uridine with no background growth on minimal medium alone.

2. Aspergillus awamori

**(i) Production of A. awamori strain UVK 143f- a Hyperproducer of Glucoamylase**

Spores of A. awamori strain NRRL 3112 were obtained after 5-7 days growth on Potato Dextrose Agar (PDA, Difco Co.) at 30°C. Spores were harvested by washing the surface of the plate with sterile 0.1% Tween-80 in distilled $H_2O$ and gently scraping the spores free. Spores were washed by centrifugation and resuspended in the same buffer to give a final concentration of between $1\times10^7$ to $2\times10^8$ spores/ml. Preparations were stored at 4°C.

Two ml of spores was added to a sterile petri plate. The top of the dish was removed and spores were exposed to an ultraviolet (UV) lamp (15 watt, germicidal). Conditions of time exposure and distance from the lamp were adjusted such that 90 to 99.9% of the spores were killed. Surviving spores were plated onto PDA medium and grown to form discrete independent colonies.

Spores from individual mutagenized colonies were inoculated into 50ml of screening media consisting of 5% corn meal, 0.5% yeast extract, 2% corn steep liquor, adjusted to pH 4.5 prior to sterilization in 250ml flasks. However, any number of media containing corn or corn starch as the carbon source would be expected to give similar results. Cultures were grown for 4-5 days at 30-35 °C with constant shaking. Samples were removed either daily or at the end of the run for assays.

Estimates of glucoamylase activity were made by measuring the release of a color producing group (para-nitro-phenol) from a colorless substrate (para-nitro-phenyl-alpha-glucoside, PNPAG).

The following protocol was utilized:

Substrate - 180mg PNPAG was dissolved in 250 ml of 0.1 M NaAcetate buffer, pH 4.7. Store at 4°C.

Assay - 1ml of substrate was equilibrated at 40°C in a water bath. 0.2ml of sample (or diluted sample) was added and incubated for 30 minutes at 40°C. 9ml of 0.1 M $Na_2CO_3$ was added with the mixture being kept at room temperature for 15 minutes for color development. The mixture was filtered through Wattman 42 filter paper and the absorbance at 420nm was read in a suitable spectrophotometer. All mutant PNPAG levels were compared to the standard amount produced by the parent strain and were reported as percent of PNPAG hyrolysis of the parent.

One glucoamylase-hyperproducing strain designated UVK 143f was selected for auxotrophic mutagenesis.

(ii) Auxotrophic Mutagenesis

Preparation of spores from A. awamori strain UVK143f, UV mutagenesis, and mutant analysis were the same as for T. reesei with the following modifications:

a. 2.5 minutes was required to give 70% killing with UV light.
b. Minimal medium was used instead of YNB agar.
c. The FOA concentration was 0.1%.

Fifteen pyrG mutants were found. Three of these isolates, designated pyr4-5, pyr4-7, and pyr4-8 were selected for transformation experiments.

**B. Transformation of A. awamori and T. reesei pyr Auxotrophs**

A. awamori and T. reesei auxotrophs were transformed by a modification of the procedure previously described for A. nidulans. Approximately 1 × $10^8$ spores were inoculated into yeast extract glucose (YEG) medium which is 2% glucose, 0.5% yeast extract supplemented with 1 mg/ml uridine. The cultures were incubated on a 37°C shaker (200 rpm) for 12 to 15 hours [T. reesei was incubated at 30°C]. Germlings were harvested by centrifugation, washed once with sterile YEG medium, then incubated at 30°C in 50% YEG medium containing 0.6 M KCl, 0.5% Novozyme 234 (Novo Industries, Denmark), 0.5% $MgSO_4 \cdot 7H_2O$, 0.05% bovine serum albumin in a sterile 200 ml plastic bottle (Corning Corp., Corning, N.Y.). After 30 minutes of shaking at 150 rpm, the protoplast suspension was vigorously pipetted up and down five times with a 10 ml pipette to disperse the clumps. The protoplast suspension was further incubated as above for one hour then filtered through sterile miracloth (Calbiochem-Behring Corp., LaJolla, California) that was wet with 0.6 M KCl. The filtered protoplasts were centrifuged, washed, and transformed with each of the plasmids pGRG1-4 as described previously.

The following modifications were made for A. awamori transformation:

1. 0.7 M KCl was used instead of 0.6 M KCl.
2. 1.2 M sorbitol was used instead of 0.6 M KCl to osmotically stabilize the transformation and regeneration medium.

**C. Analysis of A. awamori and T. reesei Transformants**

Both A. awamori and T. reesei transformants secreted chymosin polypeptides into the culture medium. This was determined by analyzing culture filtrates (results not shown) for both enzymatically active chymosin (milk clotting assay) and chymosin polypeptides that reacted with specific chymosin antibodies (enzyme immunoassays and Western immunoblotting techniques).

EXAMPLE 8

**Expression and Secretion of Heterologous Polypeptides from argB Auxotrophic Mutants of Aspergillus Species**

The expression and secretion of heterologous polypeptides from argB auxotrophs of Aspergillus species has also been achieved.

This example describes the complementary transformation of A. nidulans and A. awamori argB auxotrophs with vectors containing the argB gene from A. nidulans and DNA sequences encoding the heterologous polypeptides of plasmids pGRG1-4. The argB gene encodes ornithine transcarbamylase (OTC).

The A. nidulans argB auxotroph containing the genetic markers biA1, argB2, metG1 used herein was obtained from Dr. P. Weglenski, Department of Genetics, Warsaw University, Al. Ujazdowskie 4,00-478 Warsaw, Poland. The A. awamori argB mutant was derived as follows.

**A. Isolation of Aspergillus awamori argB Auxotrophic Mutants**

A fresh suspension of A. awamori strain UVK 143k spores was prepared and UV mutagenesis was performed as described above except that the exposure time was sufficient to kill 95% of the spores. The spores were then centrifuged, washed with sterile water, and resuspended in 25 ml of sterile minimal medium. These suspensions were incubated in a 37°C shaker with vigorous aeration. Under these conditions, wild-type spores will germinate and grow into vegetative mycelia, but auxotrophic mutants will not. The culture was aseptically filtered through sterile miracloth every six to eight hours for three days. This step removes most of the wild-type mycelia while the ungerminated auxotrophs pass through the miracloth filter (i.e., filtration enrichment). At each filtration step the filtered spores were centrifuged and resuspended in fresh minimal medium. After three days of enrichment the spores were diluted and plated on minimal agar supplemented with 50mM citrulline. The plates were incubated at 37°C for two to three days. Individual colonies were toothpicked from these plates to two screening plates -- one plate that contained minimal agar plus 10mM orihithine and one plate that contained minimal agar plus 50mM citrulline. The rationale for picking colonies to these screening plates is as follows. OTC (the argB gene product) catalyzes the conversion of ornithine to citrulline in the arginine biosynthetic pathway. Thus argB mutants (deficient in OTC) will grow on minimal medium plus citrulline but not on minimal medium with ornithine. Screening of approximately 4000 colonies by this method yielded 15 possible argB mutants. One of these strains, designated A. awamori argB3, gave no background growth on minimal medium and grew very well on minimal medium supplemented with either arginine or citrulline. Assays to determine the level of OTC activity (57) indicated that the argB3 mutant produced at least 30-fold less OTC activity than wild-type. On the basis of these data the A. awamori argB3 strain was selected for transformation experiments.

**B. Construction of argB-based Prochymosin Expression Vectors for Transformation of Aspergillus Species**

In this construction (see Figure 15) the first step was to combine the transformation enhancing sequence ANS-1 and the selectable argB gene on the same plasmid. In order to achieve this, plasmid pBB116 (59), which contains the argB gene from A. nidulans, was digested with PstI and BamHI and the indicated fragment A, which contains the argB structural gene, was isolated. Plasmid pDJB2 (59) was digested with EcoRI and PstI, and the indicated fragment B, which contains the ANS-I sequence, was isolated. In a three part ligation fragments A and B were joined together with fragment C, which contains the large EcoRI-BamHI fragment of plasmid vector pUC18 (33) to give plasmid pARG-DJB.

In the second step of this construction a synthetic DNA polylinker containing ClaI sites was inserted into pARG-DJB in order to allow the insertion of ClaI fragments which contain various prochymosin expression units. Plasmid pARG-DJB was digested with BamHI and then dephoshporylated with bacterial alkaline phosphatase. The indicated synthetic DNA polylinker was phosphorylated with T4 polynucleotide kinase, and then ligated to the cleaved pARG-DJB to give pCJl6L. Because this plasmid was found to be resistant to digestion with ClaI, it was first used to transform the E. coli dam- mutant strain GM48 in order to prevent methylation of the ClaI sites. Upon isolation of the plasmid from GM48 transformants, it was successfully cleaved with ClaI and dephosphorylated with bacterial alkaline phosphatase.

In the final step of this construciton of ClaI-cleaved pCJl6L vector was joined to each of the ClaI prochymosin expression fragments from plasmids pGRG1 through pGRG4. The resulting four plasmids, pCJ::GRG1 through pCJ::GRG4, were used to transform the argB mutants of A. nidulans and A. awamori to prototrophy. Resulting transformants were analyzed for expression of prochymosin polypeptides.

**C. Analysis of A. nidulans and A. awamori transformants**

Secreted chymosin polypeptides from A. awamori and A. nidulans transformed with pCJ::GRG1 through pCJ::GRG4 were detected by the milk clotting assay and by enzyme immunoassays and Western immunoblotting techniques. In each case (results not shown) the transformed fungi secreted biochemically active chymosin into the culture medium. oligonucleotides were 17 bases in length (17mer) and corresponded to a sequence of six amino acids (underlined in the GA1 peptide, supra) from H. grisea glucoamylase:

```
    Gln Tyr Lys Tyr Ile Lys
 5' CAA TAT AAA TAT ATT AA 3'
      G   C   G   C   C
                      A
```

The mixture of 48 oligonucleotides was synthesized in six pools, each containing eight different synthetic 17mers.

```
pool 1:   5' CAATATAAATATATTAA 3'
          G  C  G

pool 2:   5' CAATATAAATACATTAA 3'
          G  C  G

pool 3:   5' CAATATAAATATATCAA 3'
          G  C  G

pool 4:   5' CAATATAAATACATCAA 3'
          G  C  G

pool 5:   5' CAATATAAATATATAAA 3'
          G  C  G

pool 6:   5' CAATATAAATACATAAA 3'
          G  C  G
```

The oligonucleotides were synthesized on a Biosearch automated DNA synthesizer (Biosearch, San Rafael, CA) using reagents and protocols specified by the manufacturer.

4. Selection of Correct Oligonucleotide Probe

Genomic DNA from H. grisea was analyzed for the presence of glucoamylase sequences by the method of Southern (30). Briefly, H. grisea DNA was digested with BamHI restriction endonuclease. Six aliquots of this digested DNA (one for each probe pool) were fractionated according to size by electrophoresis on a 1% agarose gel. After blotting the DNA to nitrocellulose, as previously described, the DNA was fixed to the nitrocellulose filter at 80 ° C in a vacuum oven. The filter was cut into six strips, corresponding to the six aliquots of BamHI digested DNA, and each strip was hybridized for 18 hours at low stringency (2, 40) with one of the pools of synthetic oliconucleotide probes. (The probes were radiolabeled with gamma-[32P]ATP using T4 polynucleotide kinase as previously described.) After hybridization, the filters were washed 15 minutes in 2X SSC, 0.1% SDS at 37°C, and twice in 2X SSC at the same temperature. Filters were air dried, wrapped in Saran-Wrap, and applied to Kodak XOmat-AR X-ray film to obtain an autoradiographic image. After developing the autoradiogram, a faint band of hybridization corresponding to a 3.7 Kb BamHI fragment was visible from the strip that was hybridized with pool 3.

In order to improve the hybridization signal, pool 3 was re-synthesized as eight individual oligonucleotides. The Southern hybridization experiments were repeated using each of the eight oligonucleotides as probes. Only one of these 17mer probes was found to hybridize to the 3.7 Kb BamHI fragment of H. grisea genomic DNA. The sequence of the oligonucleotide was 5' CAGTACAAGTATATCAA 3'. This 17mer was used as the hybridization probe for the cloning of the H. grisea glucoamylase gene.

Although the foregoing refers to particular preferred embodiments, it will be understood that the present invention is not so limited. It will occur to those ordinarily skilled in the art that various modifications may be made to the disclosed embodiments and that such modifications are intended to be within the scope of the present invention.

The references grouped in the following bibliography and respectively cited parenthetically by number in the foregoing text, are hereby incorporated by reference.

BIBLIOGRAPHY

1. Hitzeman, R.A., et al., 1984. In Recombinant DNA Products: Insulin-Interferon-Growth Hormone, A.P. Bollon. ed., CRC Press, Boca Raton, FL; Goeddel, D.V. et al., 1979. Nature, 281: 544-548.
2. Haynes, J. et al., 1983, Nucleic Acids Res., 11:687-706; Pennica, D. et al., 1983, Nature, 301:214-221.
3. Lawn, R., et al., 1981, Nucl. Acids Res., 9:6103-6114.
4. Wood, W.I., et al., 1984, Nature, 312:330-337.
5. Heyneker, H. L. et al., European Patent Office Publication No. 0092182 published October 26, 1983.
6. Tuite, M.F., et al., 1982, EMBO J., 1:603-608.
7. Mellor, J., et al., 1982, Gene, 24:1-14.
8. Sibakov, M., et al., 1983, FEMS Microbiol Lett., 17:81-85.
9. Wells, J.A., et al., 1982, Nucl. Acids Res., 11:7911-7925.
10. Baty, D., et al., 1981, Gene, 16:79-87.
11. Sibakov, M., et al., 1984, Genetics and Biotechnology of Bacilli, A.T. Ganesan and J.A. Hoch, eds., Academic Press, NY.
12. Edens, L., et al., 1984, European Patent Office Publication No. EP0129268A2.
13. Marston, F.A.O., et al., 1984, Biotechnol., 2:800-804.
14. Foltmann, B., et al., 1979, J. Biol. Chem.. 254:8447-8456.
15. Hayenga, K.J., et al., 1984, European Patent Application EPO114507.
16. Case, M.E., et al., 1979, Proc. Natl. Acad. Sci. USA , 76:5259-5263; and Lambowitz U.S. Patent No. 4,486,533.
17. Kinsey, J.A. and J.A. Rambosek, 1984, Molecular and Cellular Biology 4:117-122.
18. Yelton, M., et al., 1984, Proc. Natl. Acad. Sci. USA , 81:1470-1474; Mullaney, E.J. et al., 1985, Mol. Gen. Genet, 199:37-45.
19. John, M.A. and J.F. Peberdy, 1984, Enzyme Microb. Technol., 6:386-389.
20. Tilburn, et al., 1982, Gene, 26:205-221.
21. Ballance, D.J., et al., 1983, Biochem. Biophys. Res. Comm. , 112:284-289.
22. Kelly, J.M. and M. Hynes, 1985, EMBO, 4:475-479.
23. Bull,J.H. and J.C. Wooton, 1984, Nature, 310:701-704.
24. Barclay, S.L. and E. Meller, 1983, Molecular and Cellular Biology , 3:2117-2130.
25. Ingolia, P.L., et al., 1984, p. 28, ASM Conf., Genet, and Mol. Bio. Ind. Microorganisms, (Abstr.)
26. Alexopoulos, C.J., 1962, Introductory Mycology, John Wiley & Sons, Inc., New York.
27. Innis, M.A., et al., 1985, Science, 228:21-26
28. Penttila, M.E., et al., 1984, Mol. Gen. Genet. , 194:494-499.
29. Shoemaker, S.P., et al., 1984, European Patent Application EPO137280A1.
30. Maniatis, T., et al., 1982, Molecular Cloning , Cold Spring Harbor Press, Cold Spring Harbor, NY.
31. Crea, R., et al., 1978, Proc. Natl. Acad. Sci. USA , 75:5765-5769.
32. Buxton, F. and Radford A., 1983, Mol. Gen. Gent., 190:403-405.
33. Yanisch-Perron, C., Vieira, J., and Messing, J., 1985, Gene, 33:103-119.
34. Clutterbuck, J.H., 1974, Aspergillus nidulans , In: Handbook of Genetics. King, R.C. ed., p. 447-510, Plenum Press, New York.
35. Towbin, H., et al., 1979, Proc. Natl. Acad. Sci. USA , 76:4350-4354.
36. Uchiyama, H., et al., 1981, J. Biochem , 90:483-487.
37. Sokol, P.A., et al., 1979, J. Clin. Microbiol. , 9:538-540.

38. Pollock, M.R.,1961, J. Gen. Microbiol. , 26:239-253.
39. Nunberg, J.H., et al., 1984, Mol. Cell. Biol. , 4:2306-2315.
40. Adelman, J.P., et al., 1983, DNA, 2:183-193.
41. Morrison, D.A. 1979, Methods Enzymol., 68:326-331.
42. Messing, J., 1983, Methods Enzymol. , 101:20-78.
43. Sanger, F., et al., 1977, Proc. Natl. Acad. Sci. USA , 74:5463-5467.
44. Bolivar, F., et al., 1977, Gene, 2:95-113.
45. Bolivar, S., 1978, Gene, 4:121-136.
46. Wallace, R.B., et al., 1981, Nucl. Acids Res. , 9:3647-3656.
47. Nunberg, J.H., et al., 1984, International Patent Publication No. W084/020921.
48. Boel, E., et al., 1984, EMBO J., 3:1581-1585.
49. European Patent Office Publication No. 0116778 published August 29, 1984.
50. Hitzeman, R.A., et al., 1983, Science, 219 :620-625.
51. Hitzeman, R.A., et al., 1983, Nucl. Acids Res. , 11:2745-2763.
52. Hitzeman, R.A., et al., 1980, J. Biol. Chem. , 255:12073.
53. Hitzeman, R.A., et al., 1982, Nucl. Acids Res. , 10:7791-7808.
54. Bech, A.M. and Foltmann, B., 1981, Neth. Milk Dairv J. , 35:275-280.
55. Foltmann, B. and Pedersen, V.B., 1977, pp. 3-22, In: Acid Proteases, J. Tang, ed., Plenum Press, NY.
56. Boeke, J.D., et al., 1984, Mol. Gen. Genet. , 197:345-346.
57. Basabe, J.R., et al., 1979, Anal. Biochem. , 94:356-360.
58. Berse, B., et al., 1983, Gene, 25:109-117.
59. Balance, D.J., et al., 1985, Gene, 36:321-331.
60. Rodriguez, H., et al., 1984, Anal. Biochem., 134:538-547.
61. Johnston, I. L., et al., 1985, EMBO J., 4:1307-1311.

## Claims

**1.** A process for making a mammalian polypeptide comprising:

transforming a filamentous fungus from the subdivision Eumycotina, with the exclusion of Saccharomyces cerevisiae, that are capable of being propagated in filamentous form with a vector which comprises a DNA sequence encoding said polypeptide, a DNA sequence encoding a signal sequence and a promoter sequence operably linked to said DNA encoding the signal sequence, said promoter sequence being functionally recognized by said filamentous fungus, whereby said DNA sequences are capable of expressing said polypeptide and of causing secretion of the polypeptide from the filamentous fungus, and expressing and secreting said polypeptide;

wherein said signal sequence is native to the mammalian polypeptide or comprises the signal sequence of bovine preprochymosin, or Mucor miehei preprocarboxy protease.

**2.** A process for making a heterologous polypeptide comprising:

transforming a filamentous fungus from the subdivision Eumycotina, with the exclusion of Saccharomyces cerevisiae, that are capable of being propagated in filamentous form with a vector which comprises a DNA sequence encoding said heterologous polypeptide, ie one that is not normally expressed and secreted in the host filamentous fungus, a DNA sequence encoding a signal sequence and a promoter sequence operably linked to said DNA encoding the signal sequence, said promoter sequence being functionally recognized by said filamentous fungus, whereby said DNA sequences are capable of expressing said polypeptide and of causing secretion of the polypeptide from the filamentous fungus, and
expressing and secreting said polypeptide;

wherein said signal sequence comprises the signal sequence of bovine preprochymosin or Mucor miehei preprocarboxy protease.

**3.** A process as claimed in claim 2 wherein the signal sequence is native to the heterologous polypeptide.

**4.** A process as claimed in claim 2 wherein the signal sequence is foreign to the heterologous polypeptide.

5. A process as claimed in claim 1 or claim 2 wherein said promoter sequence is from an endogenous gene from a filamentous fungus.

6. A process as claimed in claim 5 wherein said promoter sequence is from a filamentous fungus other than that transformed with said vector.

7. A process as claimed in claim 5 wherein said promoter comprises the promoter sequence of Aspergillus niger glucoamylase, Humicola grisea glucoamylase or Mucor miehei carboxyl protease.

8. A process as claimed in claim 1 or claim 2 further comprising a functional transcription termination and polyadenylation sequence operably linked to the DNA sequence encoding the heterologous polypeptide.

9. A process as claimed in claim 8 wherein the polyadenylation sequence comprises the polyadenylation sequence of Aspergillus niger glucoamylase or Mucor miehei carboxyl protease.

10. A process as claimed in claim 1 or claim 2 further comprising a DNA sequence encoding a selection characteristic expressible in the filamentous fungus.

11. A process as claimed in claim 10 wherein the selection characteristic is a DNA sequence encoding Neurospora crass pyr 4 , Aspergillus nidulans acetamidase, Aspergillus nidulans argB or Aspergillus nidulans trpC.

12. A process as claimed in claim 1 or claim 2 further comprising a DNA sequence capable of increasing the transformation efficiency of the vector into Aspergillus nidulans.

13. A process as claimed in claim 12 wherein the DNA sequence for increasing fungal transformation efficiency comprises ANS-1.

14. A process as claimed in claim 1 or claim 2 wherein the secreted heterologous polypeptide is an enzyme.

15. A process as claimed in claim 1 or claim 2 wherein the secreted polypeptide is biochemically active.

16. A process as claimed in claim 1 or claim 2 wherein said filamentous fungus from the subdivision Eucmycotina is selected from members of the group of genera consisting of Aspergillus. Trichoderma, Neurospora, Podospora, Endothia, Mucor, Cochibolus and Pyricularia.

17. A process as claimed in claim 1 or claim 2 wherein said filamentous fungus from the subdivision Eumycotina is selected from members of the group of genera consisting of Aspergillus and Trichoderma

18. A process as claimed in claim 1 or claim 2 wherein said filamentous fungus is selected from the group consisting of Aspergillus nidulans or Aspergillus awamori

**Patentansprüche**

1. Verfahren zum Herstellen eines Säugerpolypeptids, umfassend:

   Transformieren eines Fadenpilzes des Unterstammes Eumycotina,
   ausgenommen Saccharomyces cerevisae, die in filamentförmiger Form propagiert werden können, mit einem Vektor, umfassend eine DNA-Sequenz, die für das Polypeptid codiert, eine DNA-Sequenz, die für eine Signalsequenz codiert, und
   eine Promotorsequenz, die operativ mit der DNA verbunden ist, die für die Signalsequenz codiert, wobei die Promotorsequenz funktionell durch den Fadenpilz erkannt wird, wobei die DNA-Sequenzen das Polypeptid exprimieren und eine Sezernierung des Polypeptids aus dem Fadenpilz bewirken können, und
   Exprimieren und Sezernieren des Polypeptids;

   worin die Signalsequenz nativ für das Säugerpolypeptid ist oder die Signalsequenz von Rinder-Präprochymosin oder Mucor miehei-Präprocarboxyprotease umfasst.

**2.** Verfahren zur Herstellung eines heterologen Polypeptids, umfassend:

Transformieren eines Fadenpilzes des Unterstammes Eumycotina, ausgenommen Saccharomyces cerevisae, die in filamentförmiger Form propagiert werden können, mit einem Vektor, umfassend eine DNA-Sequenz, die für das heterologe Polypeptid codiert, d.h. für eines, das in dem Wirtsfadenpilz normalerweise nicht exprimiert und sezemiert wird, eine DNA-Sequenz, die für eine Signalsequenz codiert, und eine Promotorsequenz, die operativ mit der DNA verbunden ist, die für die Signalsequenz codiert, wobei die Promotorsequenz funktionell durch den Fadenpilz erkannt wird, wobei die DNA-Sequenzen das Polypeptid exprimieren und eine Sezemierung des Polypeptids aus dem Fadenpilz bewirken können, und Exprimieren und Sezemieren des Polypeptids;

worin die Signalsequenz die Signalsequenz von Rinder-Präprochymosin oder Mucor miehei-Präprocarboxyprotease umfasst.

**3.** Verfahren nach Anspruch 2, worin die Signalsequenz nativ für das heterologe Polypeptid ist.

**4.** Verfahren nach Anspruch 2, worin die Signalsequenz für das heterologe Polypeptid fremd ist.

**5.** Verfahren nach Anspruch 1 oder 2, worin die Promotorsequenz von einem endogenen Gen eines Fadenpilzes ist.

**6.** Verfahren nach Anspruch 5, worin die Promotorsequenz von einem Fadenpilz ist, der von dem verschieden ist, der mit dem Vektor transformiert ist.

**7.** Verfahren nach Anspruch 5, worin der Promotor die Promotorsequenz von Aspergillus niger-Glucoamylase, Humicola grises-Glucoamylase oder Mucor miehei-Carboxyl Protease umfasst.

**8.** Verfahren nach Anspruch 1 oder 2, weiterhin umfassend eine funktionelle Transkriptions-Terminierungs- und eine Polyadenylierungs-Sequenz, die operativ mit der DNA-Sequenz verbunden ist, die für das heterologe Polypeptid codiert.

**9.** Verfahren nach Anspruch 8, worin die Polyadenylierungssequenz die Polyadenylierungssequenz von Aspergillus niger-Glucoamylase oder Mucor miehei-Carboxyl Protease umfasst.

**10.** Verfahren nach Anspruch 1 oder 2, weiterhin umfassend eine DNA-Sequenz, die für ein Selektionsmerkmal codiert, das in dem Fadenpilz exprimierbar ist.

**11.** Verfahren nach Anspruch 10, worin das Selektionsmerkmal eine DNA-Sequenz ist, die für Neurospora crass pyr 4, Aspergillus nidulans Acetamidase, Aspergillus nidulans arg B oder Aspergillus nidulans trpC codiert.

**12.** Verfahren nach Anspruch 1 oder 2, weiterhin umfassend eine DNA-Sequenz, die die Transfonnationseffizienz des Vektors in Aspergillus nidulans erhöhen kann.

**13.** Verfahren nach Anspruch 12, worin die DNA-Sequenz zum Erhöhen der Pilz-Transformationseffizienz ANS-1 umfasst.

**14.** Verfahren nach Anspruch 1 oder 2, worin das sezernierte heterologe Polypeptid ein Enzym ist.

**15.** Verfahren nach Anspruch 1 oder 2, worin das sezemierte Polypeptid biochemisch aktiv ist.

**16.** Verfahren nach Anspruch 1 oder 2, worin der Fadenpilz aus dem Unterstamm Eumycotina aus Mitgliedern der Gattungsgruppe ausgewählt wird, bestehend aus Aspergillus. Trichoderma, Neurospora, Podospora. Endothia. Mucor. Cochibolus und Pyricularia.

**17.** Verfahren nach Anspruch 1 oder 2, worin der Fadenpilz aus dem Unterstamm Eumycotina ausgewählt wird aus Mitgliedem der Gattungsgruppe, bestehend aus Aspergillus und Trichoderma

**18.** Verfahren nach Anspruch 1 oder 2, worin der Fadenpilz ausgewählt wird aus der Gruppe, bestehend aus Aspergillus nidulans oder Aspergillus awamori.

## Revendications

1. procédé de production d'un polypeptide mammalien, comprenant :

   la transformation d'un champignon filamenteux de la
   subdivision des Eumycotina, à l'exclusion de *Saccharomyces cerevisiae*, qui sont capables d'être propagés sous la forme filamenteuse avec un vecteur qui comprend une séquence d'ADN codant ce polypeptide, une séquence d'ADN codant une séquence signal et une séquence promoteur liée fonctionnement à l'ADN codant la séquence signal, cette séquence promoteur étant reconnue quant à sa fonction par le champignon filamenteux concerné, si bien que les séquences d'ADN en question sont capables d'exprimer le polypeptide et de provoquer la sécrétion du polypeptide par le champignon filamenteux, et
   l'expression et la sécrétion de ce polypeptide,

   dans lequel la séquence signal est inhérente au polypeptide mammalien ou comprend la séquence signal de préprochymosine bovine ou de préprocarboxy-protéase de *Mucor miehei.*

2. Procédé de production d'un polypeptide hétérologue, comprenant :

   la transformation d'un champignon filamenteux de la subdivision des Eumycotina, à l'exclusion de *Saccharomyces cerevisiae, qui* sont capables d'être propagés sous la forme filamenteuse avec un vecteur qui comprend une séquence d'ADN codant ce polypeptide hétérologue, c'est-à-dire un polypeptide qui n'est pas normalement exprimé et sécrété dans le champignon filamenteux hôte, une séquence d'ADN codant une séquence signal et une séquence promoteur liée fonctionnellement à l'ADN codant la séquence signal, cette séquence promoteur étant reconnue quant à sa fonction par le champignon filamenteux concerné, si bien que les séquences d'ADN en question sont capables d'exprimer le polypeptide et de provoquer la sécrétion du polypeptide par le champignon filamenteux, et l'expression et la sécrétion de ce polypeptide,

   dans lequel la séquence signal comprend la séquence signal de préprochymosine bovine ou de préprocarboxy-protéase de *Mucor miehei.*

3. Procédé suivant la revendication 2, dans lequel la séquence signal est inhérente au polypeptide hétérologue.

4. Procédé suivant la revendication 2, dans lequel la séquence signal est étrangère au polypeptide hétérologue.

5. Procédé suivant la revendication 1 ou la revendication 2, dans lequel la séquence promoteur est issue d'un gène endogène provenant d'un champignon filamenteux.

6. Procédé suivant la revendication 5, dans lequel la séquence promoteur provient d'un champignon filamenteux autre que celui qui est transformé avec le vecteur indiqué.

7. Procédé suivant la revendication 5, dans lequel le promoteur comprend la séquence promoteur de glucoamylase *d'Aspergillus niger*, de glucoamylase d'*Humicola grisea* ou de carboxyl-protéase de *Mucor miehei*.

8. Procédé suivant la revendication 1 ou la revendication 2, comprenant en outre une séquence fonctionnelle de terminaison de transcription et de polyadénylation liée fonctionnellement à la séquence d'ADN codant le polypeptide hétérologue.

9. Procédé suivant la revendication 8, dans lequel la séquence de polyadénylation comprend la séquence de polyadénylation de glucoamylase *d'Aspergillus niger* ou de carboxyl-protéase de *Mucor miehei.*

10. Procédé suivant la revendication 1, ou la revendication 2, comprenant en outre une séquence d'ADN codant une caractéristique de sélection pouvant être exprimée dans le champignon filamenteux.

11. Procédé suivant la revendication 10, dans lequel la caractéristique de sélection est une séquence d'ADN codant pyr4 de *Neuropora crass,* l'acétamidase d'*Aspergillus nidulans*, argB *d'Aspergillus nidulans* ou trpC *d'Aspergillus nidulans.*

12. Procédé suivant la revendication 1 ou la revendication 2, comprenant en outre une séquence d'ADN capable d'accroître le rendement de transformation du vecteur en *Aspergillus nidulans.*

**13.** Procédé suivant la revendication 12, dans lequel la séquence d'ADN destinée à accroître le rendement de transformation fongique est ANS-1.

**14.** Procédé suivant la revendication 1 ou la revendication 2, dans lequel le polypeptide hétérologue sécrété est un enzyme.

**15.** Procédé suivant la revendication 1 ou la revendication 2, dans lequel le polypeptide sécrété est biochimiquement actif.

**16.** Procédé suivant la revendication 1 ou la revendication 2, dans lequel le champignon filamenteux de la subdivision des Eumycotina est choisi parmi les représentants du groupe des genres consistant en *Aspergillus, Trichoderma, Neurospora, Podospora, Eudorhia, Mucor, Cochibolus* et *Pyricularia.*

**17.** Procédé suivant la revendication 1 ou la revendication 2, dans lequel le champignon filamenteux de la subdivision des Eumycotina est choisi parmi les représentants du groupe des genres consistant en *Aspergillus* et *Trichoderma.*

**18.** Procédé suivant la revendication 1 ou la revendication 2, dans lequel le champignon filamenteux est choisi dans le groupe consistant en *Aspergillus nidulans* ou *Aspergillus awamori.*

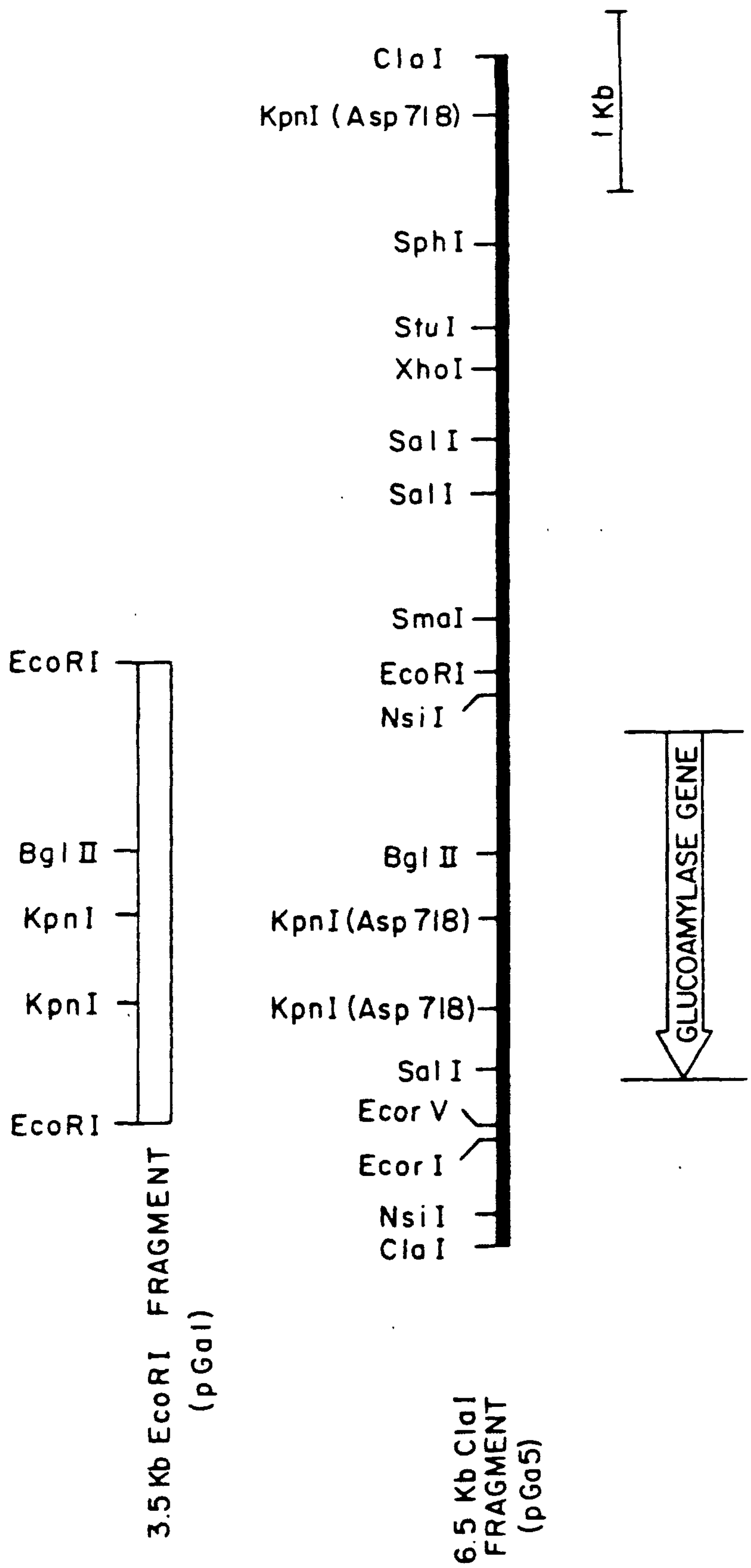
1 Kb
ClaI
KpnI (Asp 718)
SphI
StuI
XhoI
SalI
SalI
SmaI
EcoRI
NsiI
BglII
KpnI (Asp 718)
KpnI (Asp 718)
SalI
EcorV
EcorI
NsiI
ClaI
GLUCOAMYLASE GENE
EcoRI
BglII
KpnI
KpnI
EcoRI
3.5 Kb EcoRI FRAGMENT (pGa1)
6.5 Kb ClaI FRAGMENT (pGa5)

FIG.—1

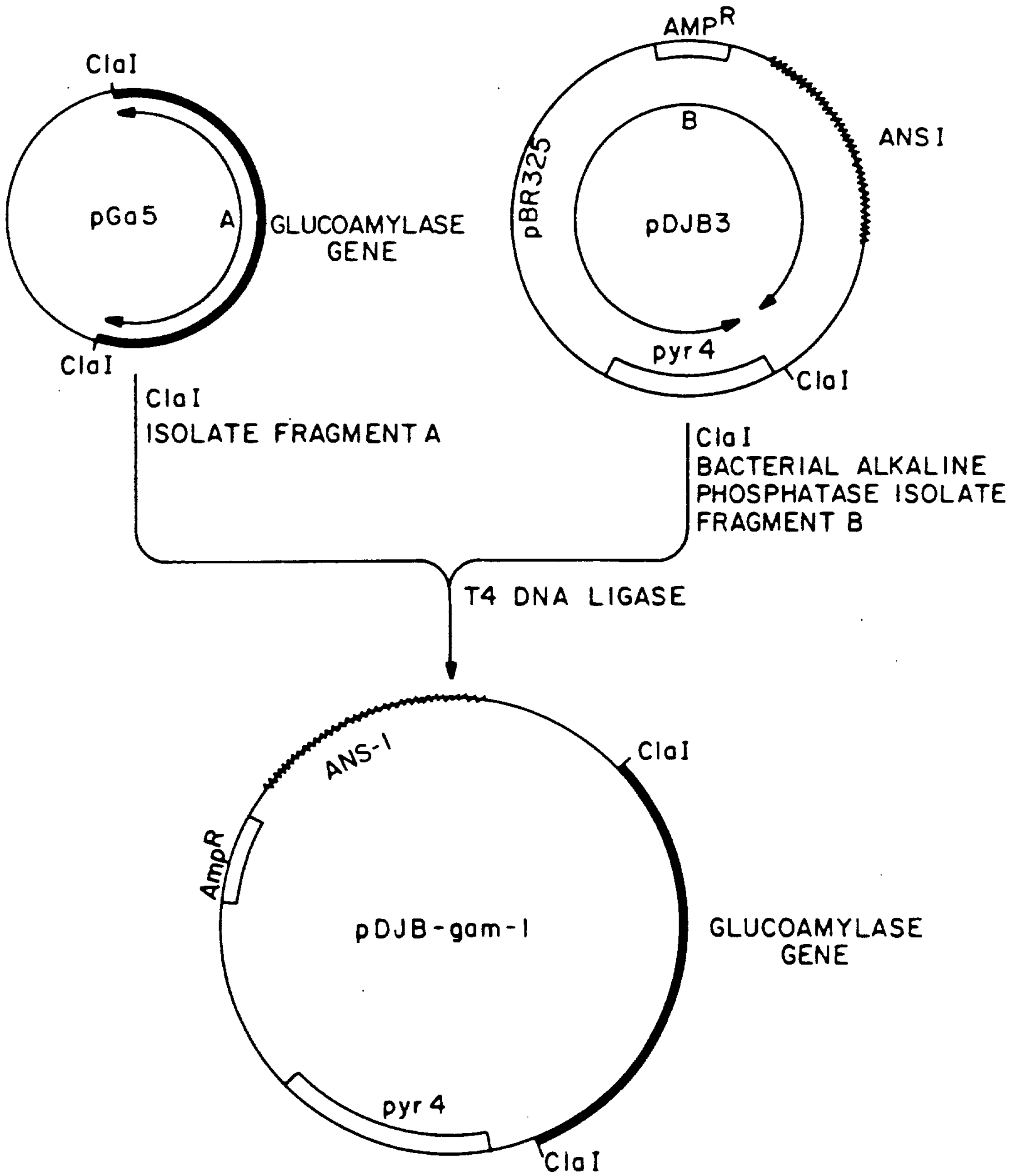
ClaI
ClaI
pGa5
A
GLUCOAMYLASE GENE
AMP^R
B
ANS I
pBR325
pDJB3
pyr 4
ClaI
ClaI
ISOLATE FRAGMENT A
ClaI
BACTERIAL ALKALINE PHOSPHATASE ISOLATE FRAGMENT B
T4 DNA LIGASE
ANS-1
ClaI
AmpR
pDJB-gam-1
GLUCOAMYLASE GENE
pyr 4
ClaI

FIG.—2

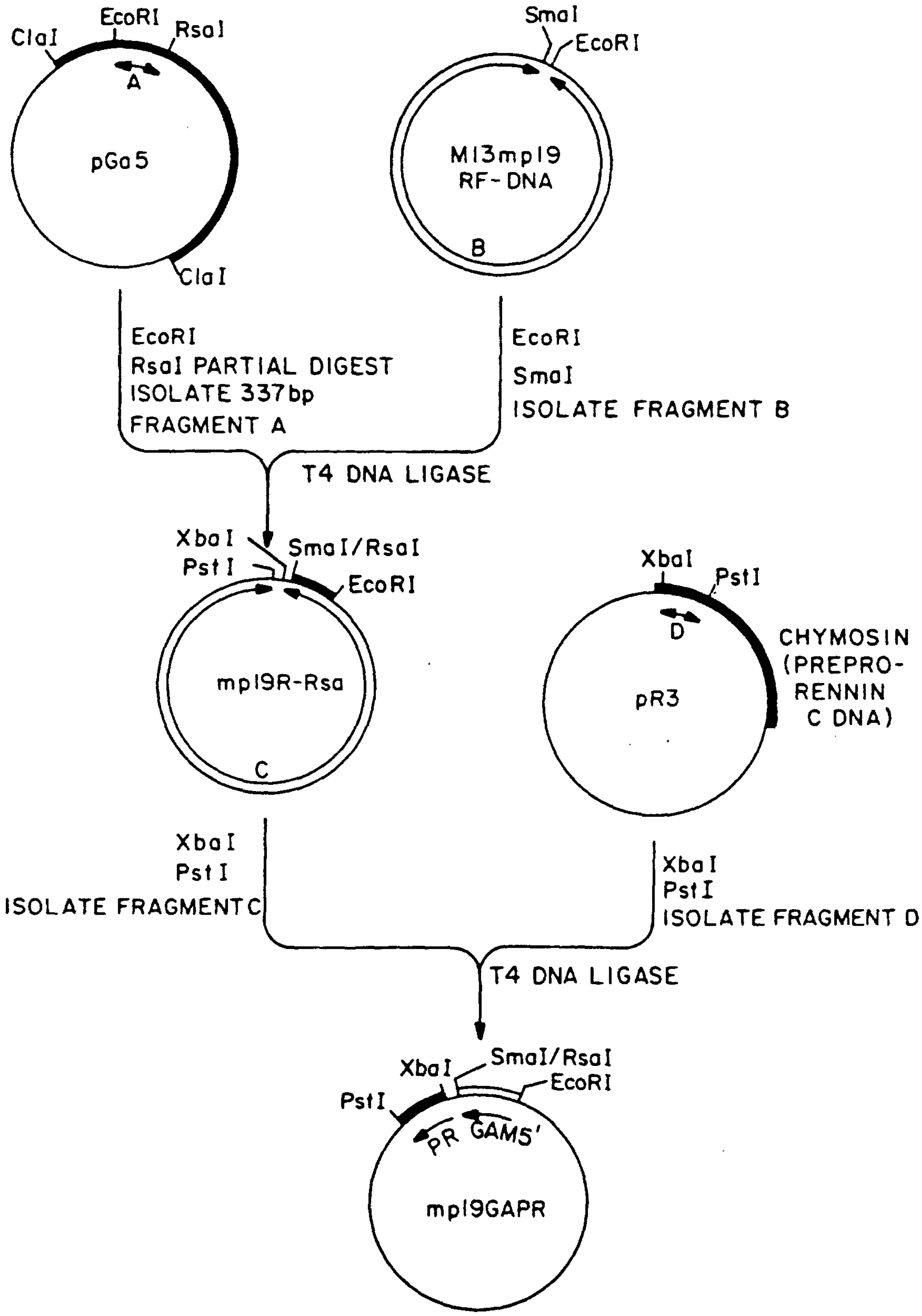
ClaI
EcoRI
RsaI
A
pGa5
ClaI
SmaI
EcoRI
M13mp19
RF-DNA
B
EcoRI
RsaI PARTIAL DIGEST
ISOLATE 337bp
FRAGMENT A
EcoRI
SmaI
ISOLATE FRAGMENT B
T4 DNA LIGASE
XbaI
PstI
SmaI/RsaI
EcoRI
mp19R-Rsa
C
XbaI
PstI
D
CHYMOSIN
(PREPRO-
RENNIN
C DNA)
pR3
XbaI
PstI
ISOLATE FRAGMENT C
XbaI
PstI
ISOLATE FRAGMENT D
T4 DNA LIGASE
XbaI
SmaI/RsaI
EcoRI
PstI
PR
GAM5'
mp19GAPR

FIG. — 3

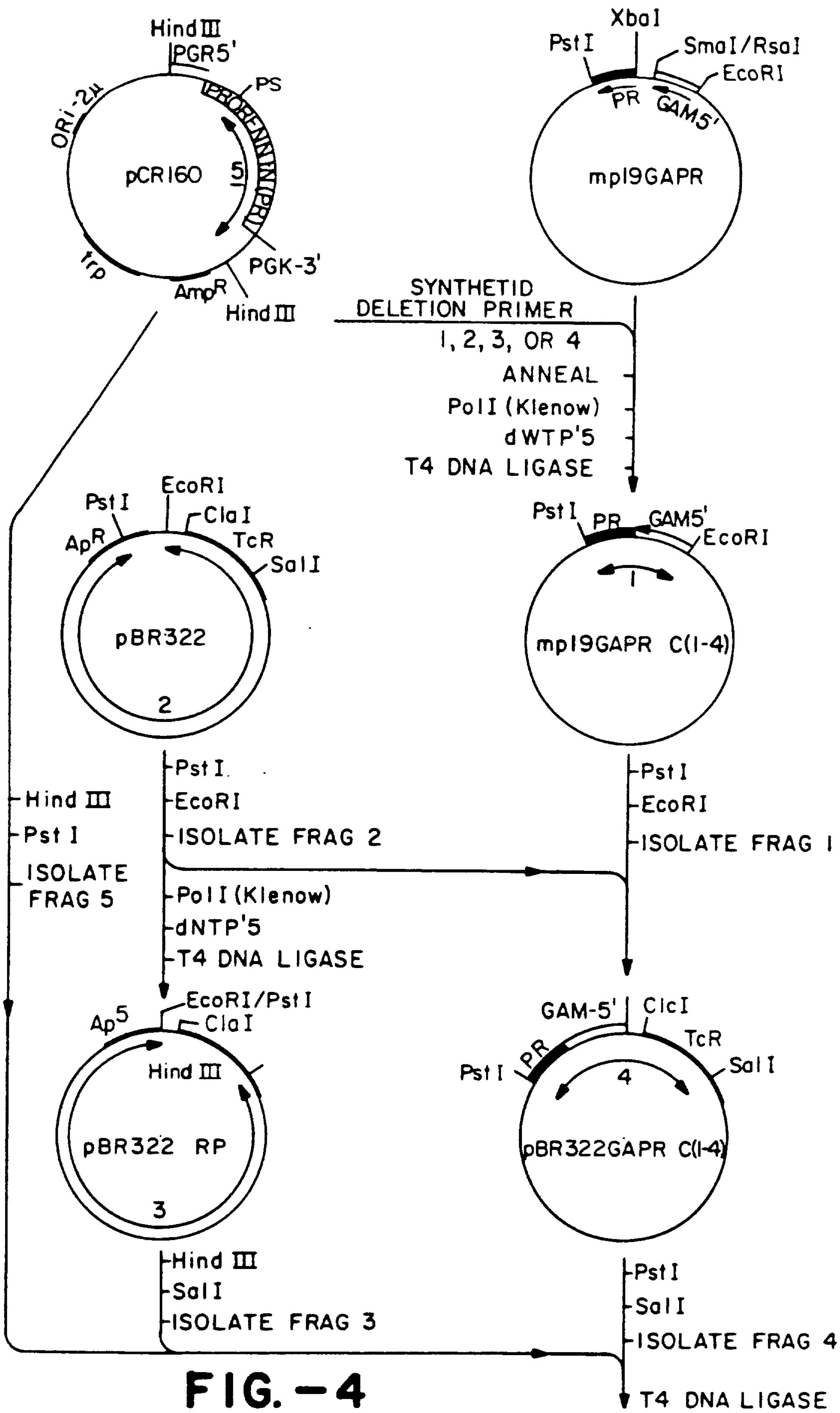

HindIII
PGR5'
PS
PROCHYMOSIN (PRO)
ORi-2μ
pCR160
5
PGK-3'
trp
AmpR
HindIII
XbaI
PstI
SmaI/RsaI
EcoRI
PR
GAM5'
mp19GAPR
SYNTHETID DELETION PRIMER
1, 2, 3, OR 4
ANNEAL
PolI (Klenow)
dWTP'5
T4 DNA LIGASE
EcoRI
PstI
ClaI
ApR
TcR
SalI
pBR322
2
PstI
PR
GAM5'
EcoRI
1
mp19GAPR C(1-4)
HindIII
PstI
ISOLATE FRAG 5
PstI
EcoRI
ISOLATE FRAG 2
PstI
EcoRI
ISOLATE FRAG 1
PolI (Klenow)
dNTP'5
T4 DNA LIGASE
EcoRI/PstI
Ap5
ClaI
HindIII
pBR322 RP
3
GAM-5'
ClcI
TcR
PstI
PR
SalI
4
pBR322GAPR C(1-4)
HindIII
SalI
ISOLATE FRAG 3
PstI
SalI
ISOLATE FRAG 4
T4 DNA LIGASE


FIG. -4

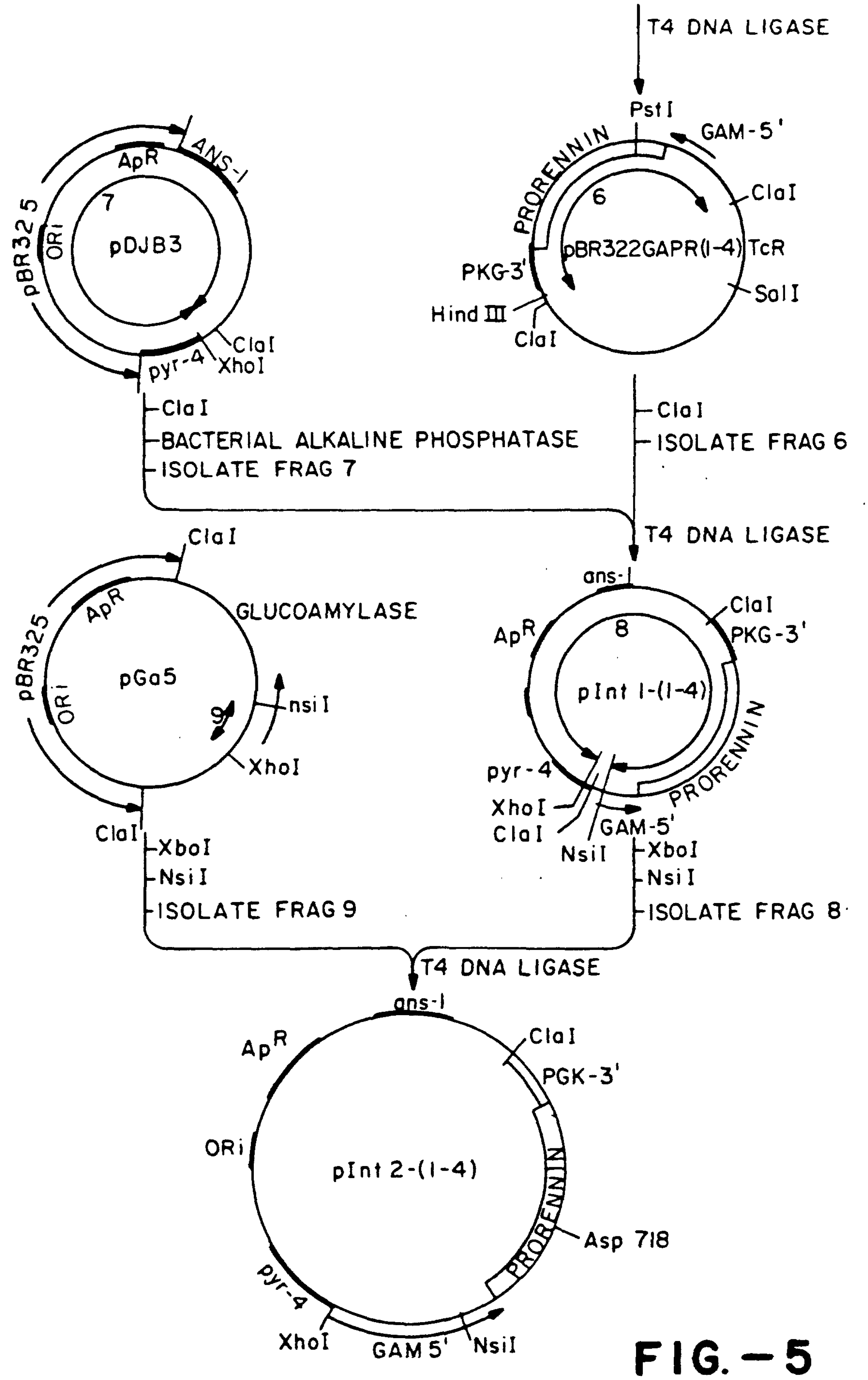
T4 DNA LIGASE
PstI
GAM-5'
PRORENNIN
6
ClaI
pBR322GAPR(1-4)
TcR
PKG-3'
SalI
Hind III
ClaI
ApR
ANS-1
7
pBR325
ORI
pDJB3
ClaI
pyr-4
XhoI
ClaI
BACTERIAL ALKALINE PHOSPHATASE
ISOLATE FRAG 7
ClaI
ISOLATE FRAG 6
T4 DNA LIGASE
ClaI
ApR
pBR325
GLUCOAMYLASE
ORI
pGa5
9
nsiI
XhoI
ClaI
XboI
NsiI
ISOLATE FRAG 9
ans-1
ClaI
ApR
8
PKG-3'
pInt 1-(1-4)
PRORENNIN
pyr-4
XhoI
ClaI
GAM-5'
NsiI
XboI
NsiI
ISOLATE FRAG 8
T4 DNA LIGASE
ans-1
ApR
ClaI
PGK-3'
ORi
pInt 2-(1-4)
PRORENNIN
Asp 718
pyr-4
XhoI
GAM 5'
NsiI

FIG.-5

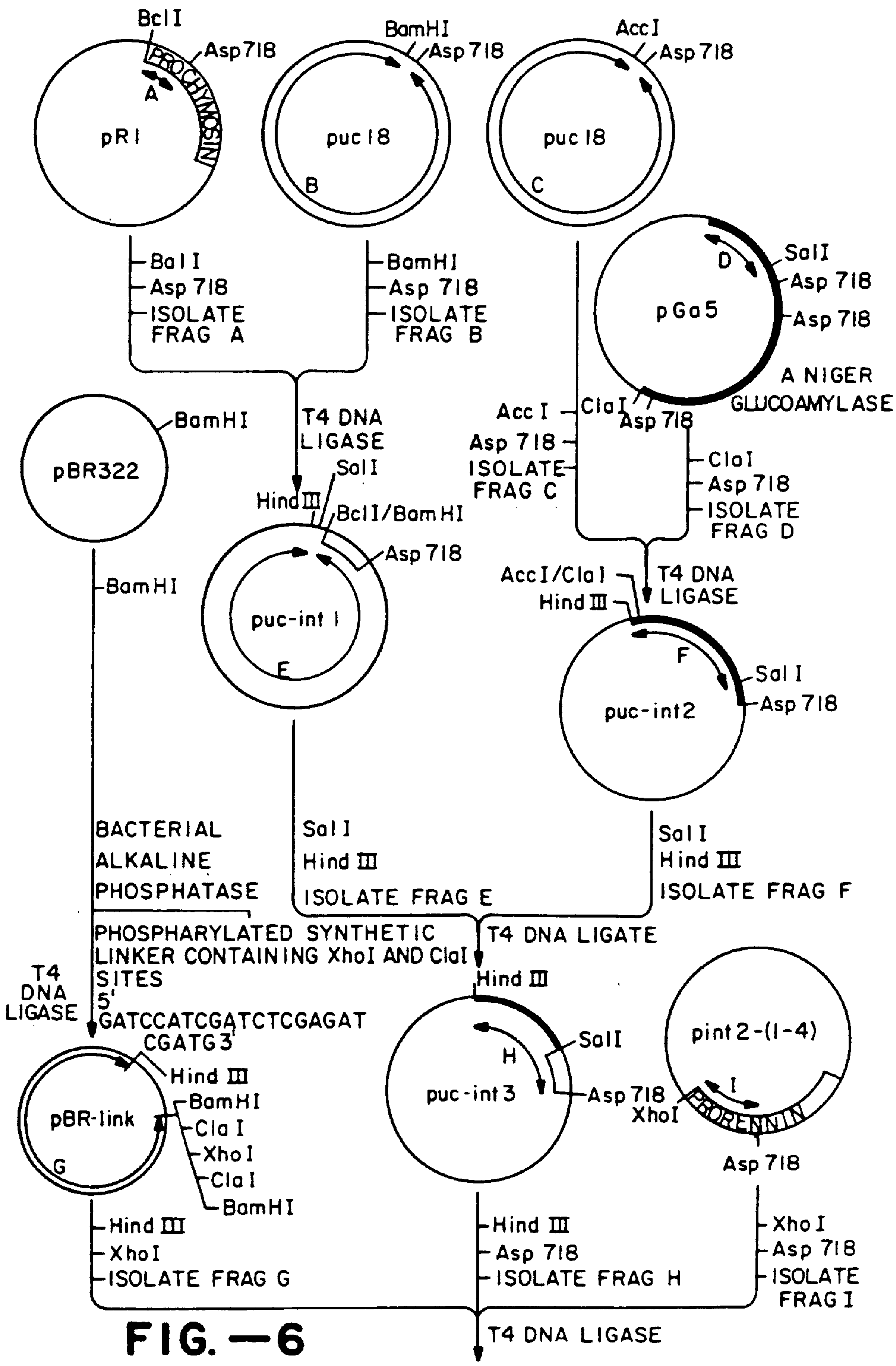
BclI
Asp718
PROCHYMOSIN
A
pRI
BamHI
Asp718
puc18
B
AccI
Asp718
puc18
C
BalI
Asp 718
ISOLATE
FRAG A
BamHI
Asp 718
ISOLATE
FRAG B
SalI
Asp 718
Asp 718
D
pGa5
A NIGER
GLUCOAMYLASE
BamHI
pBR322
T4 DNA
LIGASE
SalI
HindIII
BclI/BamHI
Asp718
puc-int I
E
AccI
ClaI
Asp 718
Asp 718
ISOLATE
FRAG C
ClaI
Asp 718
ISOLATE
FRAG D
AccI/ClaI
HindIII
T4 DNA
LIGASE
F
SalI
Asp718
puc-int2
BamHI
BACTERIAL
ALKALINE
PHOSPHATASE
SalI
HindIII
ISOLATE FRAG E
SalI
HindIII
ISOLATE FRAG F
PHOSPHARYLATED SYNTHETIC
LINKER CONTAINING XhoI AND ClaI
SITES
5'
GATCCATCGATCTCGAGAT
CGATG3'
T4
DNA
LIGASE
T4 DNA LIGATE
HindIII
SalI
H
puc-int3
Asp718
XhoI
pint2-(1-4)
I
PRORENNIN
Asp 718
HindIII
BamHI
ClaI
XhoI
ClaI
BamHI
pBR-link
G
HindIII
XhoI
ISOLATE FRAG G
HindIII
Asp 718
ISOLATE FRAG H
XhoI
Asp 718
ISOLATE
FRAG I
T4 DNA LIGASE

FIG.—6

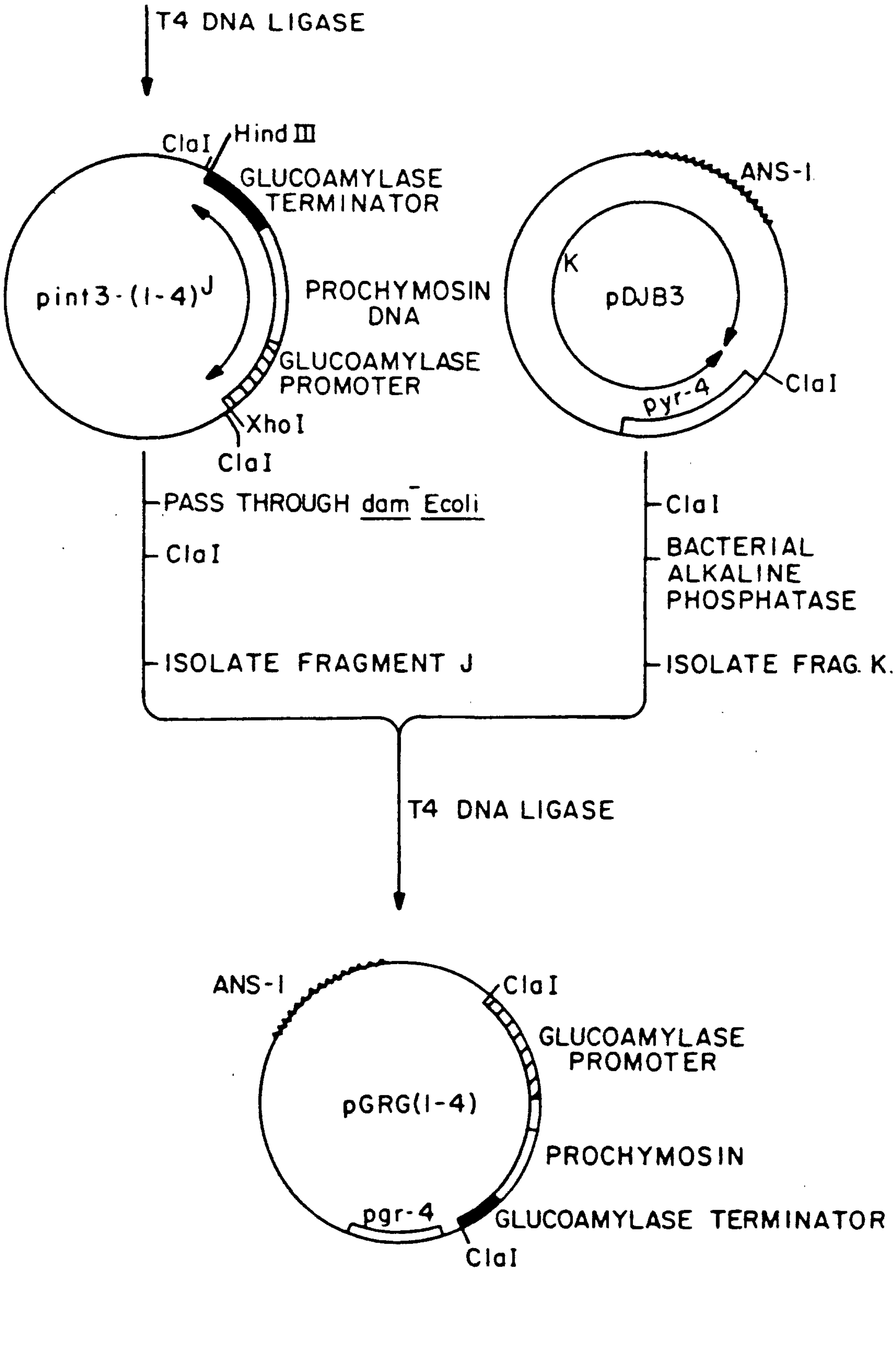
T4 DNA LIGASE
ClaI
Hind III
GLUCOAMYLASE TERMINATOR
pint3-(1-4)J
PROCHYMOSIN DNA
GLUCOAMYLASE PROMOTER
XhoI
ClaI
ANS-1
K
pDJB3
pyr-4
ClaI
PASS THROUGH dam⁻ Ecoli
ClaI
ISOLATE FRAGMENT J
ClaI
BACTERIAL ALKALINE PHOSPHATASE
ISOLATE FRAG. K.
T4 DNA LIGASE
ANS-1
ClaI
GLUCOAMYLASE PROMOTER
pGRG(1-4)
PROCHYMOSIN
pgr-4
GLUCOAMYLASE TERMINATOR
ClaI

FIG. - 7

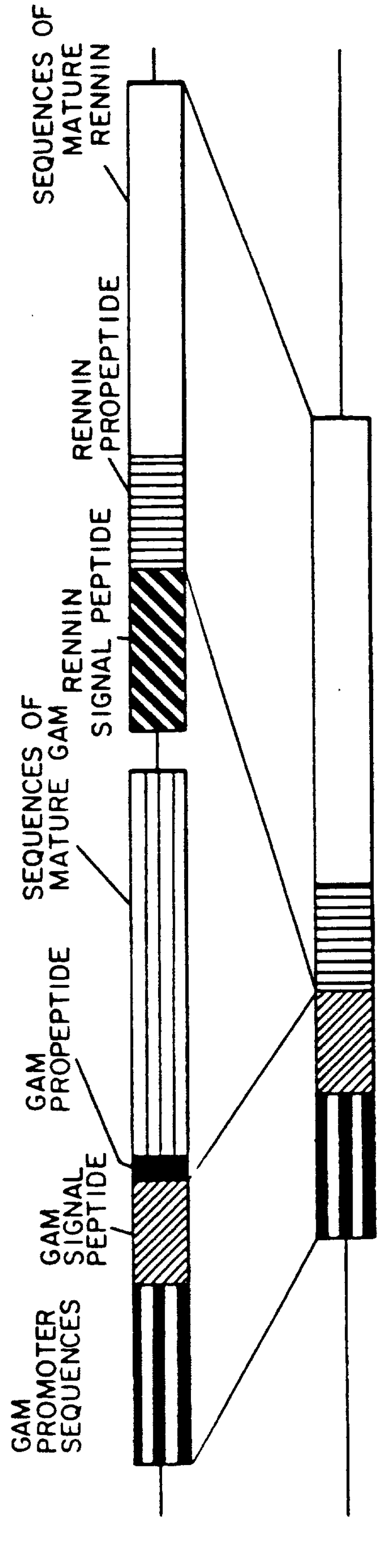


mp19GAPR C1

mpGAPR

FIG. —8A

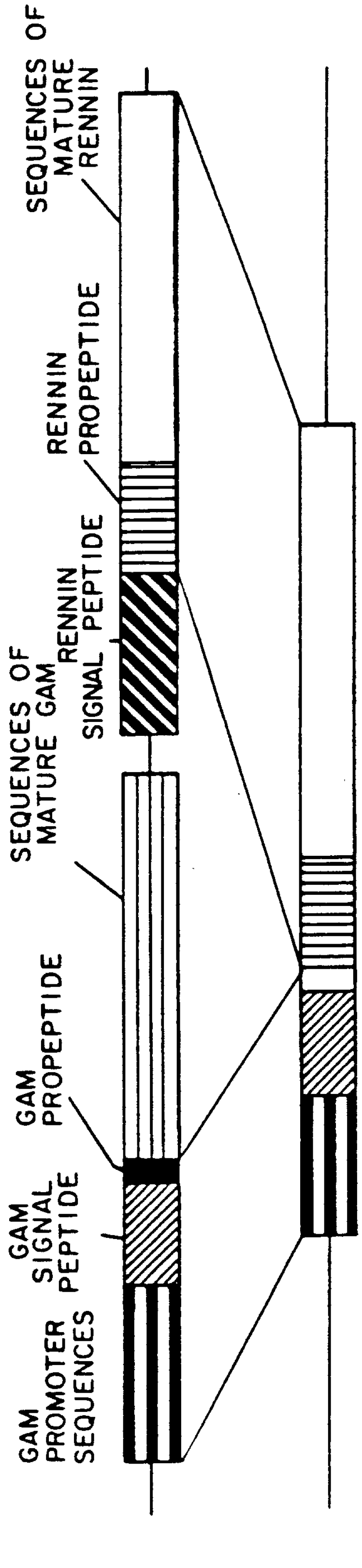


mp19GAPR C2

FIG. —8B

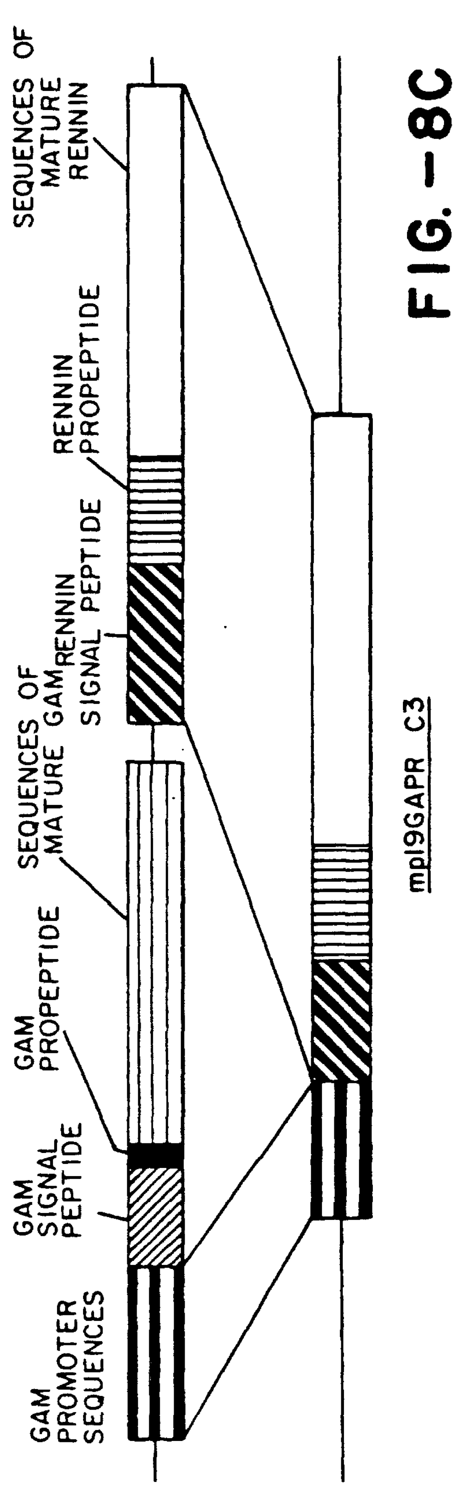
mp19GAPR
GAM PROMOTER SEQUENCES
GAM SIGNAL PEPTIDE
GAM PROPEPTIDE
SEQUENCES OF MATURE GAM
RENNIN SIGNAL PEPTIDE
RENNIN PROPEPTIDE
SEQUENCES OF MATURE RENNIN
mp19GAPR C3

FIG. -8C

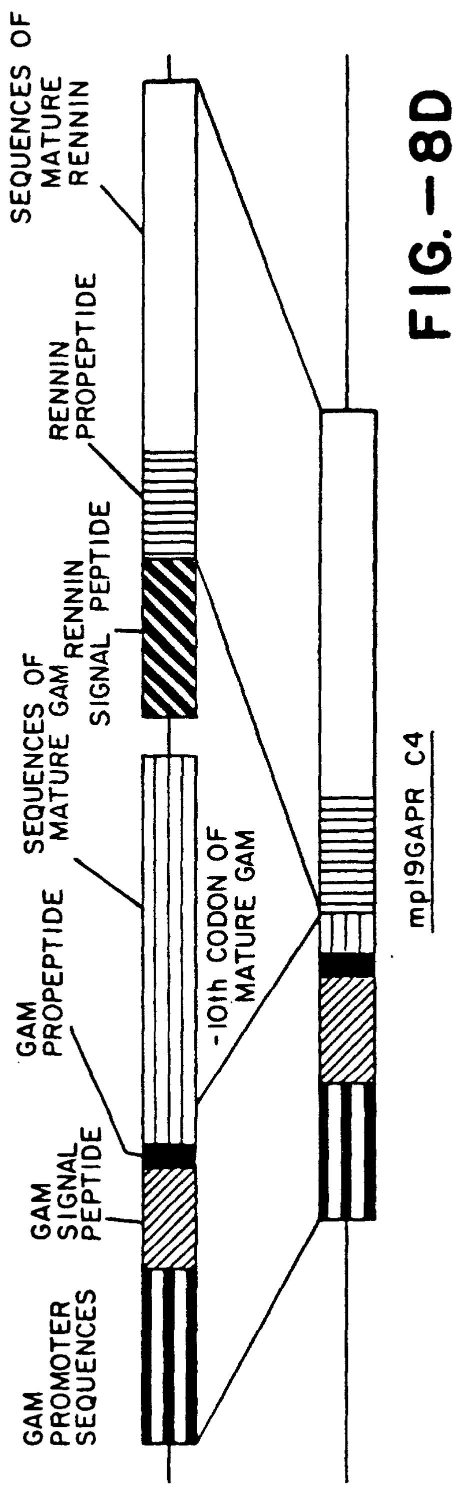
mp19GAPR
GAM PROMOTER SEQUENCES
GAM SIGNAL PEPTIDE
GAM PROPEPTIDE
SEQUENCES OF MATURE GAM
-101th CODON OF MATURE GAM
RENNIN SIGNAL PEPTIDE
RENNIN PROPEPTIDE
SEQUENCES OF MATURE RENNIN
mp19GAPR C4

FIG. -8D

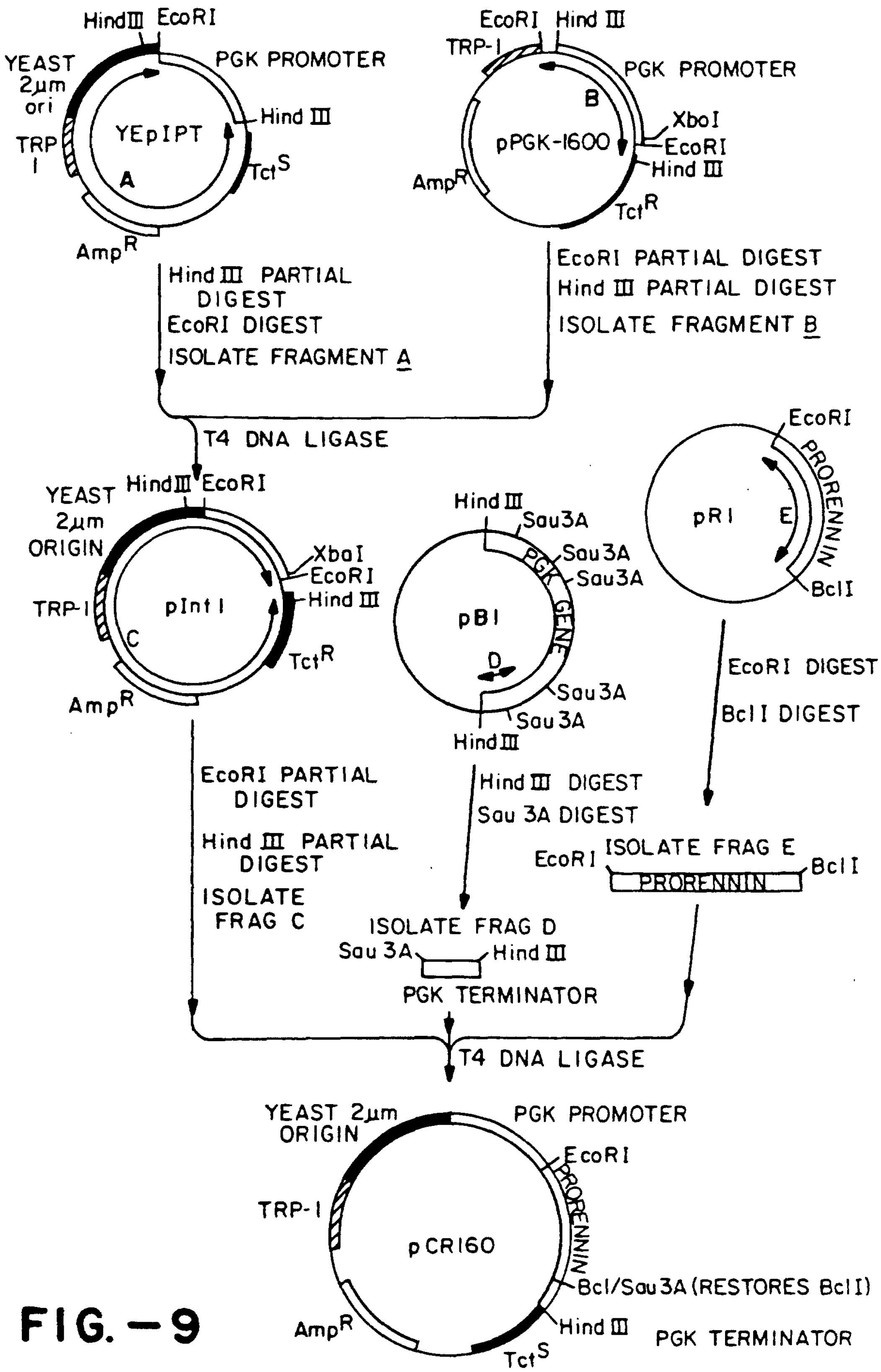
HindIII EcoRI
YEAST 2μm ori
PGK PROMOTER
Hind III
TRP I
YEpIPT
A
Tct$^S$
Amp$^R$
EcoRI Hind III
TRP-I
PGK PROMOTER
B
XbaI
EcoRI
pPGK-1600
Hind III
Amp$^R$
Tct$^R$
Hind III PARTIAL DIGEST
EcoRI DIGEST
ISOLATE FRAGMENT A
EcoRI PARTIAL DIGEST
Hind III PARTIAL DIGEST
ISOLATE FRAGMENT B
T4 DNA LIGASE
EcoRI
PRORENNIN
pRI
E
BclI
YEAST 2μm ORIGIN
HindIII EcoRI
XbaI
EcoRI
Hind III
TRP-I
pInt I
C
Tct$^R$
Amp$^R$
Hind III
Sau3A
Sau3A
Sau3A
PGK GENE
pBI
D
Sau3A
Sau3A
Hind III
EcoRI DIGEST
BclI DIGEST
EcoRI PARTIAL DIGEST
Hind III PARTIAL DIGEST
ISOLATE FRAG C
Hind III DIGEST
Sau 3A DIGEST
EcoRI
ISOLATE FRAG E
BclI
PRORENNIN
ISOLATE FRAG D
Sau 3A
Hind III
PGK TERMINATOR
T4 DNA LIGASE
YEAST 2μm ORIGIN
PGK PROMOTER
EcoRI
PRORENNIN
TRP-I
pCRI60
Bcl/Sau3A (RESTORES BclI)
Amp$^R$
Hind III
PGK TERMINATOR
Tct$^S$

FIG. — 9

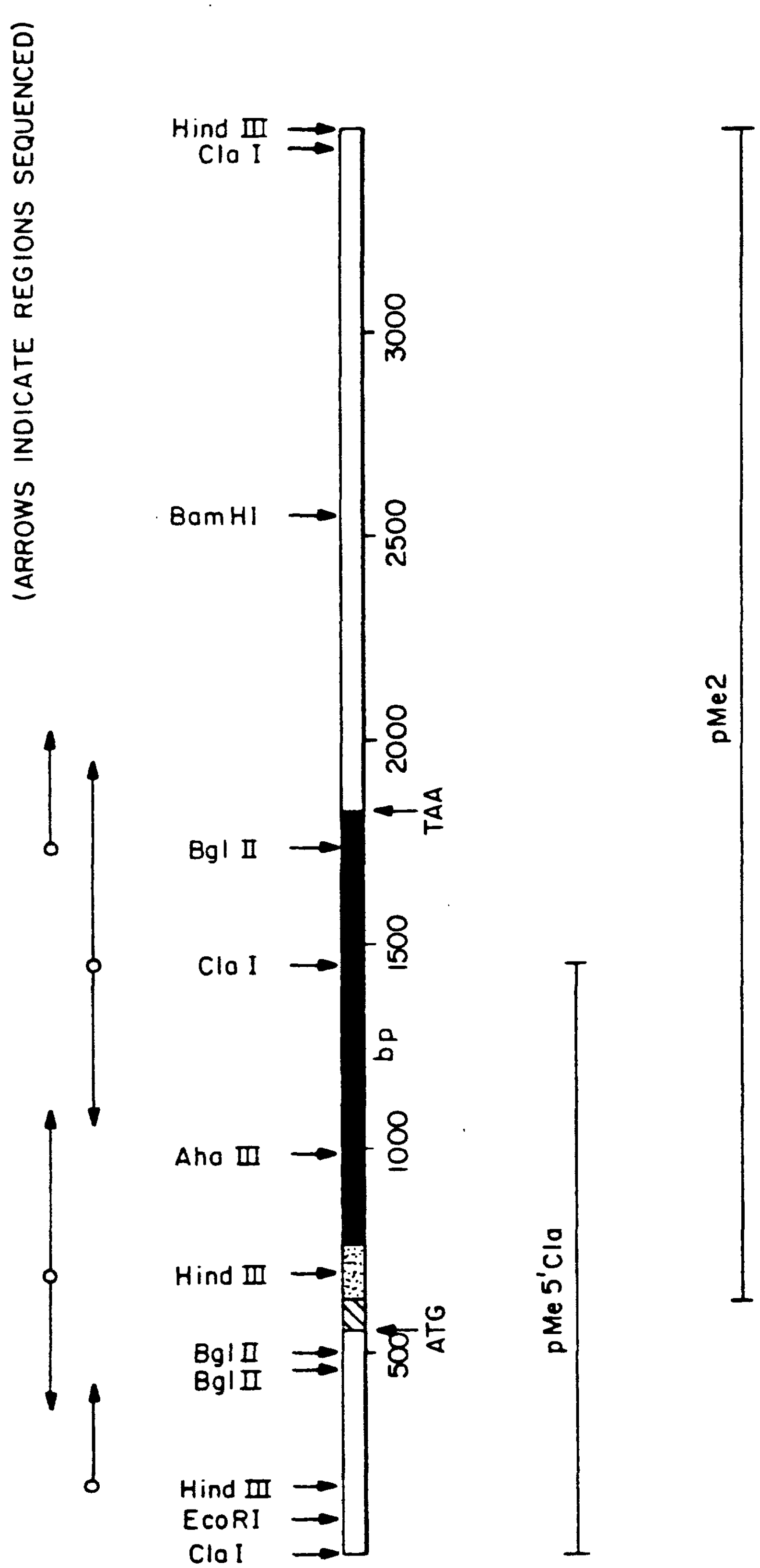
(ARROWS INDICATE REGIONS SEQUENCED)
Hind III
Cla I
BamHI
Bgl II
Cla I
Aha III
Hind III
Bgl II
Bgl II
Hind III
EcoRI
Cla I
3000
2500
2000
TAA
1500
bp
1000
500
ATG
pMe 5'Cla
pMe2

FIG. – 10

length: 1900

```
  1 AAGCTTCCAT TTCAGAAAAG AAAGCTCTCG GAGTTGTCAT TGAGGCATTT TTACAGCCTT

 61 TTTCCGCTCT CACTCTGTAT TTCTATGAGA AAGAGAAAGA TAGGAATGTC ATGCATAAGA

121 TGCTACAAGA TCATGCCAAC GGTAGTACAC ACAATTCCTG CCTTTTATGA CCTTTTTATG

181 CTAAGTCTTT TTGGAAATTT ATCCCGCGGT CCTAGGTTGT ACCCAGTCGT TGCATCCACC

241 TTTCCTGGTA AAAACATACA ATTATGGACT AAATGGTTTC TTAAATGTCA ACACTCTAGA

301 CGGAAGGGCA CAAAGATCTT TAGTGCCTGG GCCAACTGTA GGTAGATCTT TTTTTCATAT

361 AAAACCAGAC GAGTGTGAAG GTTGCGAGAC TCCATCAGAT TCCGACC ATG CTC TTC
                                                    Met Leu Phe

417 TCT CAG ATT ACT TCT GCG ATC CTT TTA ACA GCG GCT TCC TTG TCG CTT
                        -61                                     -51
    Ser Gln Ile Thr Ser Ala Ile Leu Leu Thr Ala Ala Ser Leu Ser Leu

465 ACC ACT GCT CGC CCG GTA TCC AAG CAA TCC GAG TCC AAG GAC AAG CTT
                                        -41
    Thr Thr Ala Arg Pro Val Ser Lys Gln Ser Glu Ser Lys Asp Lys Leu

513 CTG GCG CTT CCT CTC ACC TCG GTG TCC CGC AAG TTC TCT CAA ACC AAG
                -31                                     -21
    Leu Ala Leu Pro Leu Thr Ser Val Ser Arg Lys Phe Ser Gln Thr Lys

561 TTC GGT CAG CAA CAA CTT GCT GAG AAG CTA GCA GGT CTC AAG CCC TTC
                                -11
    Phe Gly Gln Gln Gln Leu Ala Glu Lys Leu Ala Gly Leu Lys Pro Phe

609 TCT GAA GCT GCC GCA GAC GGC TCC GTC GAT ACG CCC GGC TAT TAC GAC
         -1  1                                   11
    Ser Glu Ala Ala Ala Asp Gly Ser Val Asp Thr Pro Gly Tyr Tyr Asp

657 TTT GAT CTG GAG GAG TAT GCT ATT CCG GTC TCC ATT GGT ACT CCT GGT
                            21
    Phe Asp Leu Glu Glu Tyr Ala Ile Pro Val Ser Ile Gly Thr Pro Gly
```

FIG.—11A

```
705  CAA GAC TTT TTG CTC TTG TTC GAC ACT GGC AGC TCC GAT ACT TGG GTT
     31                                      41
     Gln Asp Phe Leu Leu Leu Phe Asp Thr Gly Ser Ser Asp Thr Trp Val

753  CCA CAC AAG GGT TGC ACC AAG TCT GAA GGT TGT GTT GGC AGC CGA TTC
                     51                                      61
     Pro His Lys Gly Cys Thr Lys Ser Glu Gly Cys Val Gly Ser Arg Phe

801  TTT GAT CCA TCG GCT TCC TCC ACT TTT AAA GCA ACT AAC TAC AAC CTA
                                     71
     Phe Asp Pro Ser Ala Ser Ser Thr Phe Lys Ala Thr Asn Tyr Asn Leu

849  AAC ATC ACC TAC GGT ACT GGC GGC GCA AAC GGT CTT TAC TTT GAA GAC
             81                                      91
     Asn Ile Thr Tyr Gly Thr Gly Gly Ala Asn Gly Leu Tyr Phe Glu Asp

897  AGC ATC GCT ATC GGC GAC ATC ACC GTG ACC AAG CAA ATT CTG GCT TAC
                             101
     Ser Ile Ala Ile Gly Asp Ile Thr Val Thr Lys Gln Ile Leu Ala Tyr

945  GTC GAT AAT GTT CGC GGC CCA ACT GCT GAG CAG TCT CCT AAC GCT GAC
     111                                     121
     Val Asp Asn Val Arg Gly Pro Thr Ala Glu Gln Ser Pro Asn Ala Asp

993  ATT TTC CTT GAT GGT CTC TTT GGT GCA GCC TAC CCA GAC AAC ACG GCC
                     131                                     141
     Ile Phe Leu Asp Gly Leu Phe Gly Ala Ala Tyr Pro Asp Asn Thr Ala

1041 ATG GAA GCA GAG TAT GGA TCG ACT TAT AAC ACT GTT CAC GTC AAC CTC
                                     151
     Met Glu Ala Glu Tyr Gly Ser Thr Tyr Asn Thr Val His Val Asn Leu

1089 TAC AAG CAA GGC TTG ATC TCT TCT CCT CTT TTC TCG GTC TAC ATG AAC
             161                                     171
     Tyr Lys Gln Gly Leu Ile Ser Ser Pro Leu Phe Ser Val Tyr Met Asn

1137 ACT AAC AGC GGC ACT GGA GAG GTC GTC TTT GGT GGA GTC AAC AAC ACG
                             181
     Thr Asn Ser Gly Thr Gly Glu Val Val Phe Gly Gly Val Asn Asn Thr

1185 CTT CTC GGC GGC GAC ATT GCC TAC ACG GAC GTT ATG AGT CGT TAT GGT
     191                                     201
     Leu Leu Gly Gly Asp Ile Ala Tyr Thr Asp Val Met Ser Arg Tyr Gly

1233 GGT TAT TAC TTC TGG GAC GCA CCC GTC ACA GGT ATC ACC GTC GAT GGA
                     211                                     221
     Gly Tyr Tyr Phe Trp Asp Ala Pro Val Thr Gly Ile Thr Val Asp Gly
```

FIG.—11B

```
1281 TCT GCT GCT GTC AGG TTC TCG AGA CCC CAA GCA TTC ACC ATC GAT ACT
                                 231
     Ser Ala Ala Val Arg Phe Ser Arg Pro Gln Ala Phe Thr Ile Asp Thr

1329 GGC ACC AAC TTT TTC ATT ATG CCC TCA AGC GCC GCT TCT AAG ATT GTC
         241                                 251
     Gly Thr Asn Phe Phe Ile Met Pro Ser Ser Ala Ala Ser Lys Ile Val

1377 AAA GCA GCT CTC CCT GAT GCC ACT GAA ACC CAG CAG GGC TGG GTT GTT
                             261
     Lys Ala Ala Leu Pro Asp Ala Thr Glu Thr Gln Gln Gly Trp Val Val

1425 CCT TGC GCT AGC TAC CAG AAC TCC AAG TCG ACT ATC AGC ATC GTC ATG
     271                                 281
     Pro Cys Ala Ser Tyr Gln Asn Ser Lys Ser Thr Ile Ser Ile Val Met

1473 CAA AAG TCC GGC TCA AGC AGT GAC ACT ATT GAG ATC TCG GTT CCT GTC
                     291                                 301
     Gln Lys Ser Gly Ser Ser Ser Asp Thr Ile Glu Ile Ser Val Pro Val

1521 AGC AAA ATG CTT CTT CCA GTC GAC CAA TCG AAC GAG ACT TGC ATG TTT
                                 311
     Ser Lys Met Leu Leu Pro Val Asp Gln Ser Asn Glu Thr Cys Met Phe

1569 ATC ATT CTT CCC GAC GGT GGT AAC CAG TAC ATT GTT GGC AAC TTG TTC
             321                                 331
     Ile Ile Leu Pro Asp Gly Gly Asn Gln Tyr Ile Val Gly Asn Leu Phe

1617 CTG CGC TTC TTT GTC AAT GTT TAC GAC TTT GGC AAC AAC CGT ATC GGC
                             341
     Leu Arg Phe Phe Val Asn Val Tyr Asp Phe Gly Asn Asn Arg Ile Gly

1665 TTT GCA CCT TTG GCC TCG GCT TAT GAA AAC GAG TAA       AGGGGCACCA A
     351                                 361
     Phe Ala Pro Leu Ala Ser Ala Tyr Glu Asn Glu OC*

1712 TTCTTCTTT AGCTGCTCAG ATAACTTTGT AACTCTCTGA TATACTCTTT ATAACCTTTA T

1772 TTCTCACTT TTTAACTGTA TTCCAATACA TTATTTGAAC TTACTAAATA TTACGCTTAT T

1832 CTTGTTTGG GTGAGTTGTA GAGTAAAAAA AATGCTTAGA AGCGGAATTG TATTTTGCAA G

1892 GAATGTACA
```

FIG.—11C

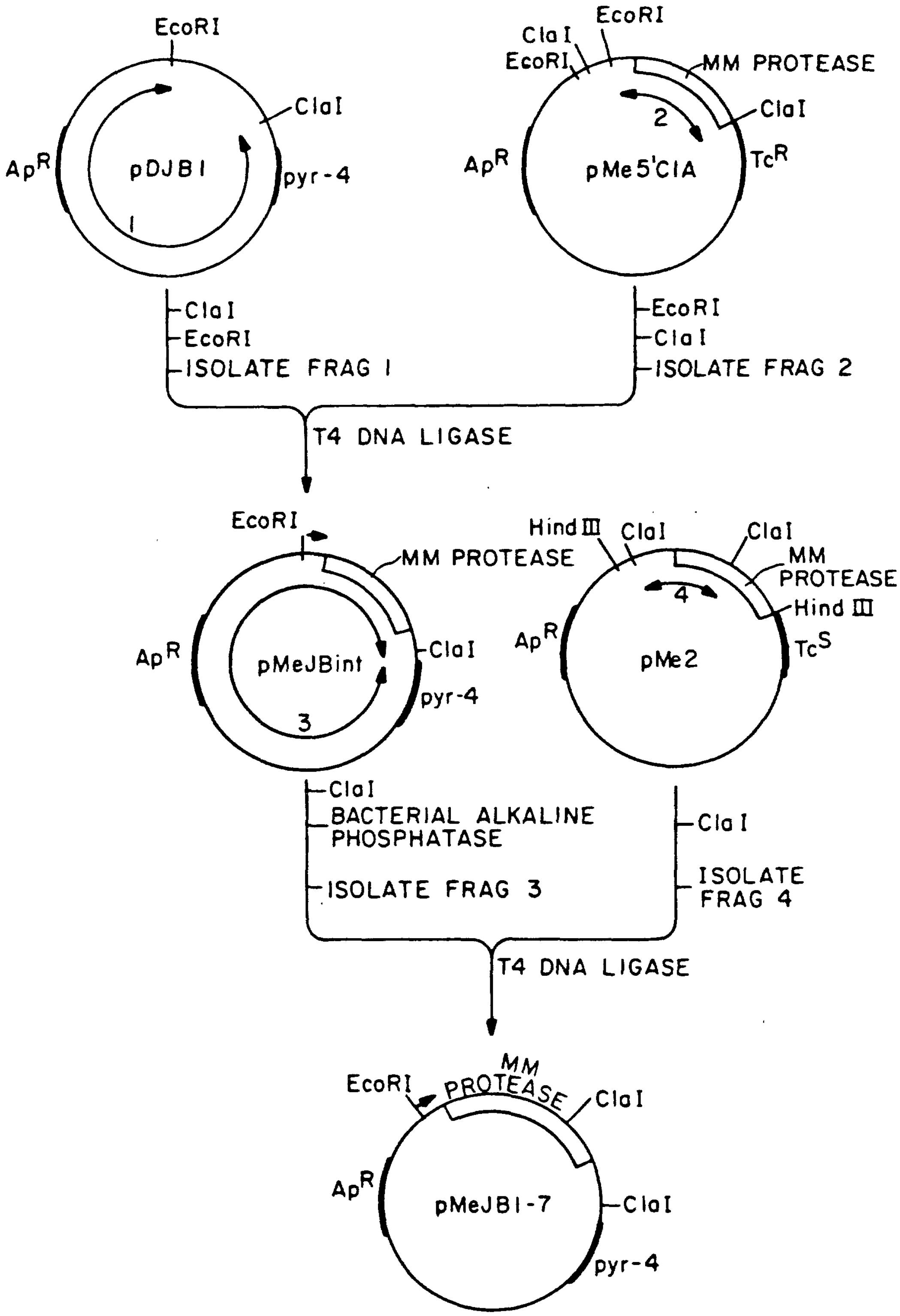
EcoRI
ClaI
ApR
pDJB1
pyr-4
1
EcoRI
ClaI
EcoRI
MM PROTEASE
ClaI
2
ApR
pMe5'ClA
TcR
ClaI
EcoRI
ISOLATE FRAG 1
EcoRI
ClaI
ISOLATE FRAG 2
T4 DNA LIGASE
EcoRI
MM PROTEASE
ClaI
ApR
pMeJBint
pyr-4
3
HindIII
ClaI
ClaI
MM PROTEASE
4
Hind III
ApR
pMe2
TcS
ClaI
BACTERIAL ALKALINE PHOSPHATASE
ISOLATE FRAG 3
ClaI
ISOLATE FRAG 4
T4 DNA LIGASE
MM PROTEASE
EcoRI
ClaI
ApR
pMeJB1-7
ClaI
pyr-4

FIG. - 12

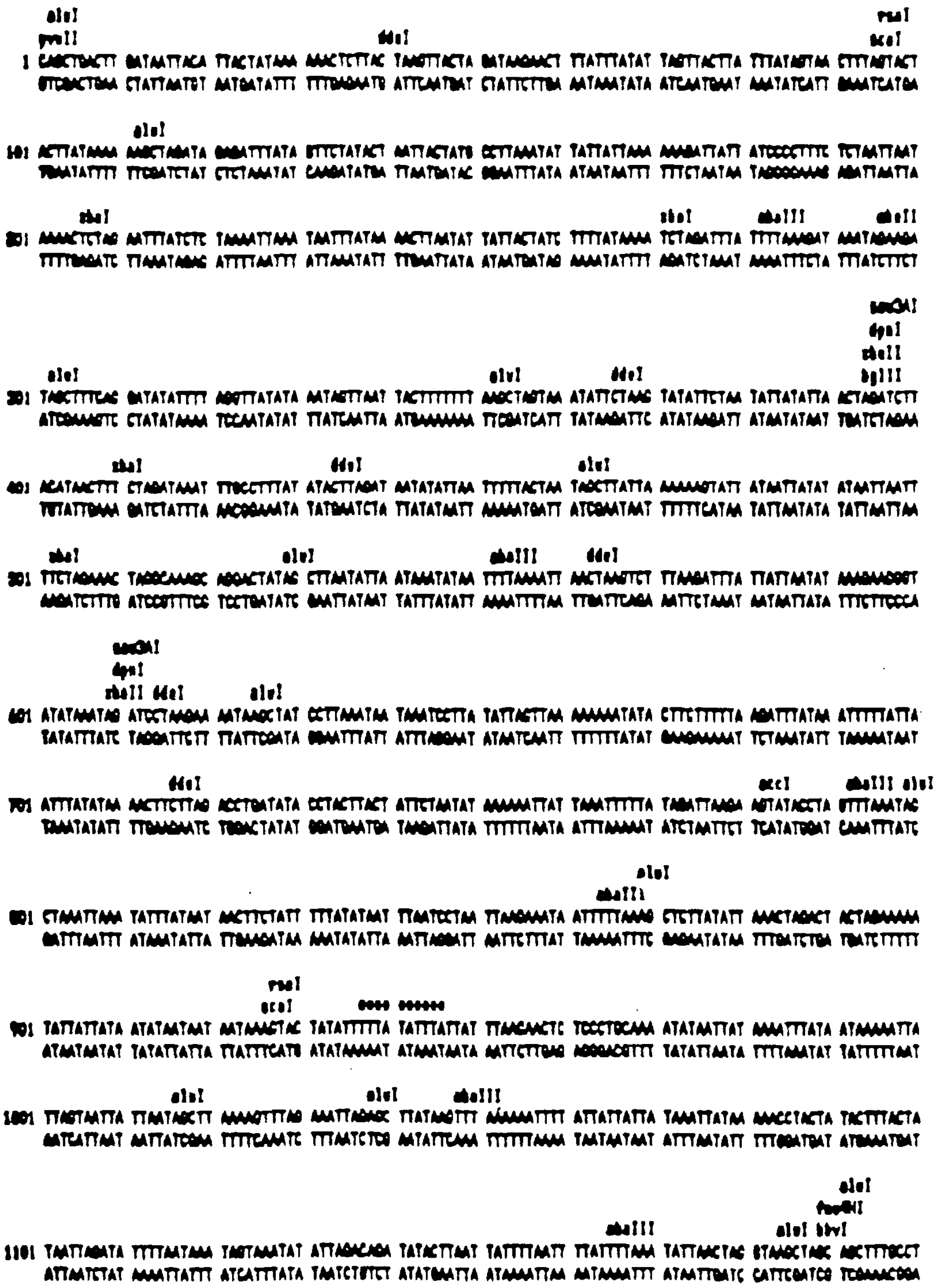

FIG.—13A

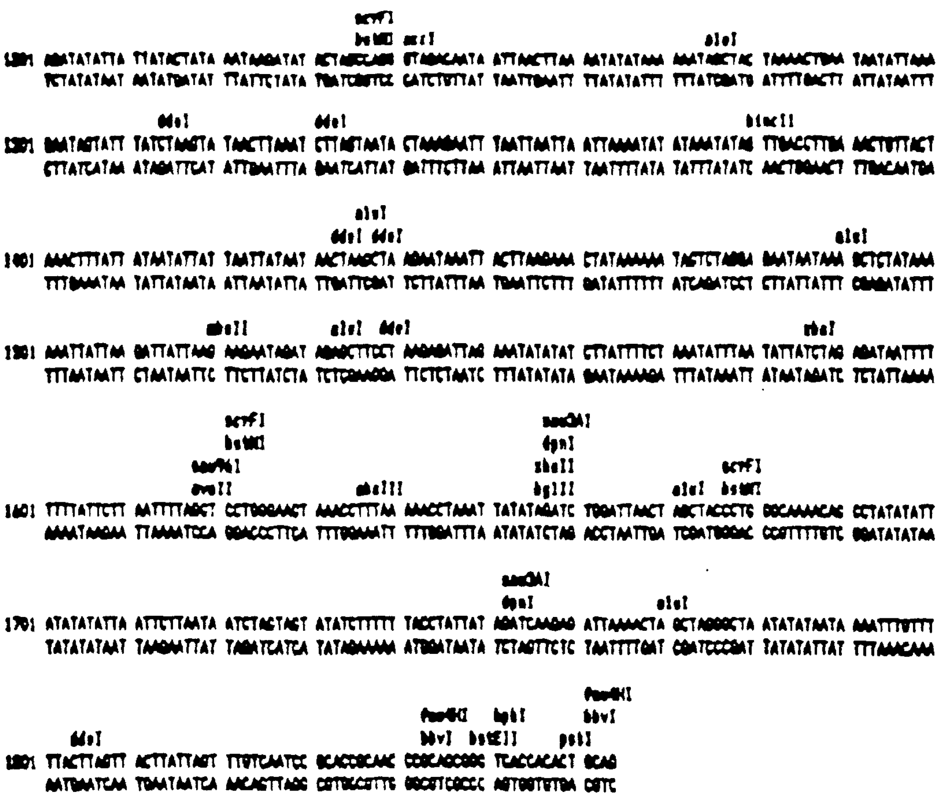

FIG.—13B

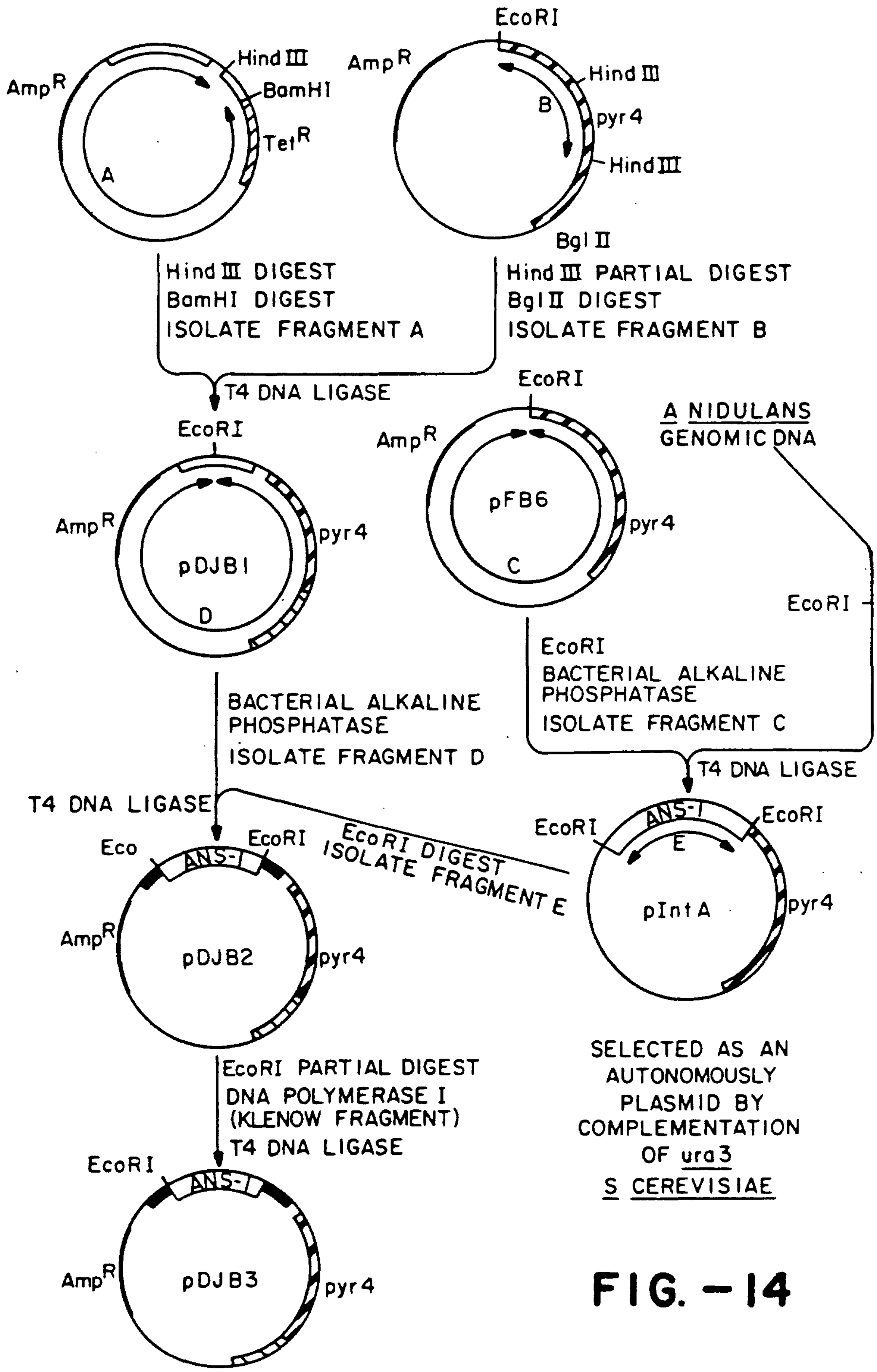
EcoRI
AmpR
HindIII
BamHI
TetR
A
AmpR
HindIII
B
pyr4
HindIII
BglII
HindIII DIGEST
BamHI DIGEST
ISOLATE FRAGMENT A
HindIII PARTIAL DIGEST
BglII DIGEST
ISOLATE FRAGMENT B
T4 DNA LIGASE
EcoRI
EcoRI
AmpR
A NIDULANS
GENOMIC DNA
AmpR
pDJB1
D
pyr4
pFB6
C
pyr4
EcoRI
EcoRI
BACTERIAL ALKALINE
PHOSPHATASE
ISOLATE FRAGMENT C
BACTERIAL ALKALINE
PHOSPHATASE
ISOLATE FRAGMENT D
T4 DNA LIGASE
T4 DNA LIGASE
EcoRI DIGEST
ISOLATE FRAGMENT E
Eco
EcoRI
ANS-1
AmpR
pDJB2
pyr4
EcoRI
ANS-1
EcoRI
E
pIntA
pyr4
EcoRI PARTIAL DIGEST
DNA POLYMERASE I
(KLENOW FRAGMENT)
T4 DNA LIGASE
SELECTED AS AN
AUTONOMOUSLY
PLASMID BY
COMPLEMENTATION
OF ura3
S CEREVISIAE
EcoRI
ANS-1
AmpR
pDJB3
pyr4

FIG. - 14

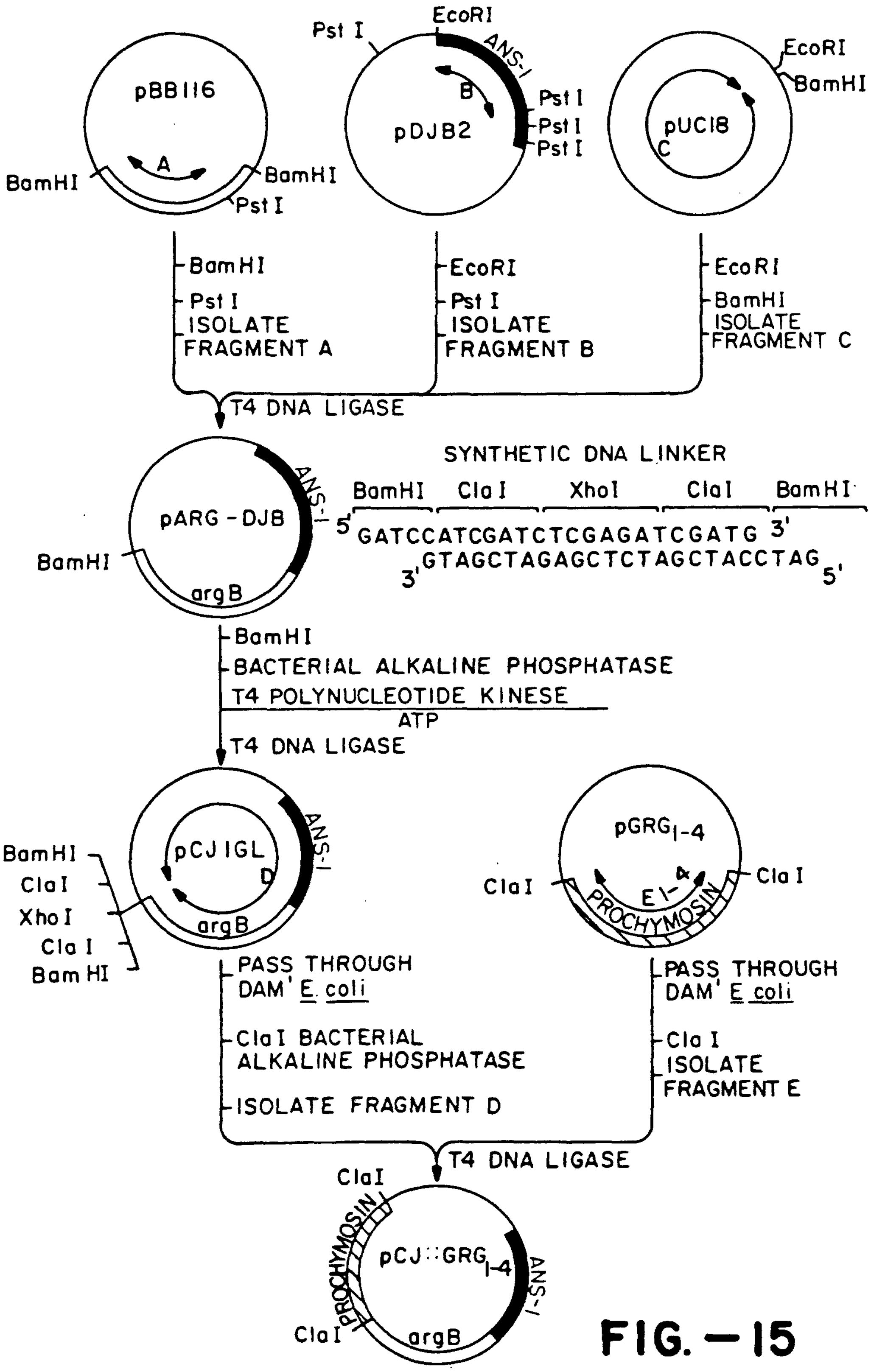
pBB116
A
BamHI
BamHI
PstI
pDJB2
PstI
EcoRI
ANS-I
B
PstI
PstI
PstI
pUC18
C
EcoRI
BamHI
BamHI
PstI
ISOLATE
FRAGMENT A
EcoRI
PstI
ISOLATE
FRAGMENT B
EcoRI
BamHI
ISOLATE
FRAGMENT C
T4 DNA LIGASE
pARG-DJB
ANS-I
BamHI
argB
SYNTHETIC DNA LINKER
BamHI ClaI XhoI ClaI BamHI
5' GATCCATCGATCTCGAGATCGATG 3'
3' GTAGCTAGAGCTCTAGCTACCTAG 5'
BamHI
BACTERIAL ALKALINE PHOSPHATASE
T4 POLYNUCLEOTIDE KINESE
ATP
T4 DNA LIGASE
pCJ1GL
D
ANS-I
argB
BamHI
ClaI
XhoI
ClaI
BamHI
pGRG1-4
ClaI
ClaI
E1-4
PROCHYMOSIN
PASS THROUGH
DAM' E. coli
ClaI BACTERIAL
ALKALINE PHOSPHATASE
ISOLATE FRAGMENT D
PASS THROUGH
DAM' E. coli
ClaI
ISOLATE
FRAGMENT E
T4 DNA LIGASE
ClaI
PROCHYMOSIN
pCJ::GRG1-4
ANS-I
ClaI
argB

FIG. — 15